(19) Europäisches Patentamt
European Patent Office
Office européen des brevets

(11) **EP 4 685 154 A1**

(12) **EUROPEAN PATENT APPLICATION**

(43) Date of publication:
**28.01.2026 Bulletin 2026/05**

(21) Application number: **24190365.7**

(22) Date of filing: **23.07.2024**

(51) International Patent Classification (IPC):
***C07K 14/435*** *(2006.01)* ***A61P 7/02*** *(2006.01)*
***A61K 38/17*** *(2006.01)*

(52) Cooperative Patent Classification (CPC):
**A61P 7/02; A61F 2/00; A61K 38/1767; C07K 14/43536; C07K 14/815; C12N 9/1044; C12Y 203/02013;** C07K 2319/00; G01N 2333/43504

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Rheinische Friedrich-Wilhelms-Universität Bonn**
**53113 Bonn (DE)**

(72) Inventors:
- **Imhof, Diana**
  **53113 Bonn (DE)**
- **Kühl, Toni**
  **53113 Bonn (DE)**
- **Singh, Sneha**
  **53113 Bonn (DE)**
- **Biswas, Arijit**
  **53113 Bonn (DE)**

(74) Representative: **Jacobi, Markus Alexander**
**Patentanwälte**
**Isenbruck Bösl Hörschler PartG mbB**
**Eastsite One**
**Seckenheimer Landstrasse 4**
**68163 Mannheim (DE)**

Remarks:
The complete document including Reference Table(s) and the Sequence Listing(s) can be downloaded from the EPO website

(54) **POLYPEPTIDE DERIVED FROM TRIDEGIN**

(57) The invention relates to a polypeptide derived from natural tridegin by structural modification, which may serve as an inhibitor of plasma coagulation factor XIIIa, and to pharmaceutical compositions comprising such polypeptide. The present invention further refers to such polypeptide or pharmaceutical composition for use in a method for treating or preventing a thrombotic event in a patient. Moreover, the invention relates to an endoprosthesis or an enzyme-linked immunosorbent assay (ELISA) plate coated with a polypeptide of the present invention.

EP 4 685 154 A1

## Description

The present invention relates to a polypeptide derived from natural tridegin by structural modification, which may serve as an inhibitor of plasma coagulation factor XIII, and to pharmaceutical compositions comprising such polypeptide. The present invention further refers to such polypeptide or pharmaceutical composition for use in a method for treating or preventing a thrombotic event in a patient. Moreover, the invention relates to an endoprosthesis or an enzyme-linked immunosorbent assay (ELISA) plate coated with a polypeptide of the present invention.

Thrombotic events can still be health-threatening and even life-threatening pathological conditions. For many years, cardiovascular diseases which are often associated with thrombotic events, including ischemic stroke and venous thromboembolism, have been the most common cause of death in Germany and worldwide (Germany 2020: 34.3%). The formation of thrombosis, which is due to a combination of dysregulated hemostasis, changes in vascular functions and changes in blood flow velocity, play an essential role in the development of such diseases.

The development of anticoagulants for the prophylaxis and treatment of thromboembolic diseases therefore remains the focus of research, although there are some known anticoagulants. In the recent past, an interest has been on compounds that specifically switch off a specific coagulation factor, such as thrombin (FIIa) and FXa inhibitors, in contrast to earlier, non-specific therapy principles such as the use of vitamin K antagonists or heparins.

Compounds targeting the coagulation factors may play a major role in monitoring and treatment of excessive coagulation processes, and the focus in drug development is still on natural products, including venoms from various organisms, such as snakes and bees as well as saliva from leeches.

Substances isolated from leeches have been used in antithrombotic therapy for thousands of years, with the enzymatic and non-enzymatic reactions of the blood coagulation cascade and the factors involved playing a major role in the regulation and specific control of these processes. This may include cysteine-rich, anticoagulant peptides isolated from leeches. In particular, compounds targeting the serine protease thrombin have already been successfully used as lead structures for the development of the now used active inhibitors bivalirudin and desirudin.

In contrast to other blood coagulation factors, only a few useful inhibitors have been described for blood coagulation factor XIII (FXIIIa). In this context, fibrin polymerization starts with the cleavage of fibrinogen into fibrin that is then finally cross-linked by FXIIIa. The catalytic mechanism of FXIIIa includes a distinct three-dimensional structure of the active site with the catalytic triad Cys314, His373, and Asp396.

A technical drawback of many FXIIIa inhibitors is that many of these interact with a low potency towards FXIIIa and are non-specific, i.e. they simultaneously inhibit other enzymes such as the tissue transglutaminase TG2. It has been tried to prepare polypeptide inhibitors of FXIIIa, which contain a number of disulfide bonds in the 1990s (WO 96/34890). One of the very few if not the only polypeptide that has a selective inhibitory effect on FXIIIa is tridegin (Finney et al., Biochemical Journal, 1997, 324:797-805; Wallis et al., Blood Coagulation and Fibrinolysis, 1997, 8:291-295; Seale et al., Thrombosis and Haemostasis, 1997, 77(5):959-963).

Tridegin is a polypeptide of 66 amino acids having different disulfide bond connectivities (cf. Böhm et al., Journal of Medicinal Chemistry, 2014, 57:10355-10365). In the wild-type form, the tridegin may optionally form a triple disulfidebridged polypeptide. Tridegin was isolated from the saliva of the leech *Haementeria ghilianii* in 1997 and has since been recognized as one of the most potent, naturally occurring FXIIIa inhibitors (Finney et al., Biochemical Journal, 1997, 324:797-805; Wallis et al., Blood Coagulation and Fibrinolysis, 1997, 8:291-295; Seale et al., Thrombosis and Haemostasis, 1997, 77(5):959-963). Compared to other FXIIIa inhibitors, tridegin bears the technical advantage that it inhibits FXIIIa efficiently, while at the same time affecting other enzymes such as the transglutaminase TG2 to a considerably and desirable low extent. Tridegin has a high potency against FXIIIa (which may be IC50 = 10 nM) and is considered as being rather specific, as it has essentially no inhibitory effect against other coagulation factors (thrombin, FXa) and cysteine proteases (e.g. bromelain, papain) (Finney et al., Biochemical Journal, 1997, 324:797-805).

Tridegin is considered having a 23-fold weaker inhibitory effect against the transglutaminase TG2 compared to FXIIIa. Tridegin is considered as a specific reversible peptide inhibitor of factor XIIIa in the last step of the blood coagulation cascade.

In view of the technical advantages, tridegin has been considered as an interesting pharmacological tool and potential drug for treating or preventing thrombotic events, including addressing certain cardiovascular diseases.

However, native tridegin also bears non-negligible technical drawbacks. Access to larger contents and purity of tridegin from natural resources native is hampered.

Truncated polypeptides with a focus of the C-terminal parts partly corresponding to tridegin have been described in US-A 2009/0226415 and Böhm et al., Journal of Medicinal Chemistry, 2014, 57:10355-10365. Such truncated forms are, however, less active. The heterologous expression or synthetic provision of tridegin faces severe challenges. The tridegin having three disulfide bridges bears 15 conformational isomers (Böhm et al., Journal of Medicinal Chemistry, 2014, 57:10355-10365, Bäuml et al., Journal of Medicinal Chemistry, 2019, 62:3513-3523; Bäuml et al., European Journal of Medicinal Chemistry, 2020, 201:112474). Thus, the preparation of tridegin is severely complicated due to the multiple disulfide bonds and consequently the excessive yield loss associated with it.

Furthermore, it has been tried to prepare tridegin derivatives having only two disulfide bonds (Bäuml et al., Journal of Medicinal Chemistry, 2019, 62:3513-3523; Schmitz et al., International Journal of Molecular Sciences, 2021, 22:880). This, however, bears the technical drawback that still undesirable complex synthesis steps are needed and structural isomers by the generation of different disulfide bonds and re-arrangements of disulfide bonds ("shuffling") may occur.

It has been tried to replace cysteine residues by serine residues to observe the impact of the disulfide bonds on the three-dimensional structure and inhibitory activity (Schmitz et al., International Journal of Molecular Sciences, 2021, 22:880). The synthesis of such a linear tridegin analogue was greatly simplified, the yield was enhanced and the loss of inhibitory activity was seen, but in an acceptable range (Böhm et al., Journal of Medicinal Chemistry, 2014, 57:10355-10365, Bäuml et al., Journal of Medicinal Chemistry, 2019, 62:3513-3523), while - at the same time - specificity for FXIIIa was kept.

Therefore, there was an unmet need for polypeptides that bear a sufficient inhibitory effect on FXIIIa that can be prepared more efficiently and have a desirably good inhibitory effect on and specificity for FXIIIa as well as proteolytic stability.

Surprisingly, it has been found that a polypeptide as claimed can also be very well used as inhibitor of FXIIIa and as agent usable for treating and/or preventing a thrombotic event in a patient or as an endoprosthesis in biomedical applications.

A first aspect relates to a polypeptide comprising a polypeptide strand of SEQ ID NO: 1:

$$X^{1}LLPX^{5}X^{6}EWHQGIPNPX^{16}X^{17}WX^{19}GADLEX^{25}AQDQYX^{31}AFIPQX^{37}X^{38}P X^{40}SELIX^{45}PMDDIYQX^{53}PVEFPNLPLX^{63}PX^{65}E,$$

wherein amino acid moieties $X^{19}$ and $X^{25}$ are each independently selected from the group consisting of Ala and Cys(Me);

wherein amino acid moieties $X^1$, $X^6$, $X^{16}$, $X^{38}$, $X^{40}$, $X^{45}$, $X^{53}$, $X^{63}$, and $X^{65}$ are each independently selected from the group consisting of Lys, D-Lys, Arg, D-Arg, Orn, and Cit;

wherein amino acid moieties $X^5$, $X^{17}$, $X^{31}$, and $X^{37}$ are each independently selected from the group consisting of Ser, Ala, Cys(Me), an amino acid moiety forming a lactam bond with another one of the amino acid moieties selected from the group consisting of $X^5$, $X^{17}$, $X^{31}$, and $X^{37}$, and Cys optionally forming a disulfide bond with another Cys at an amino acid moiety selected from the group consisting of $X^5$, $X^{17}$, $X^{31}$, and $X^{37}$;

wherein not more than two of $X^5$, $X^{17}$, $X^{31}$, and $X^{37}$ are Cys,

wherein optionally one or more of the L-amino acid moieties may be replaced by the respective one or more D-amino acids,

or a retro-inverso analogue of the sequence thereof,

or a peptidomimetic analogue of the sequence thereof,

or a pharmaceutical salt thereof.

It was found that such a polypeptide could be feasibly prepared and that such a polypeptide bears beneficial properties on inhibiting FXIIIa.

The polypeptides of the present invention are comparably stable against proteolytic degradation while at the same time maintaining their inhibitory activity and enabling comparably easier production and purification protocols. These may additionally show superior behavior concerning physicochemical features and pharmacokinetics.

The polypeptide of the present invention may serve as a specific pharmacological inhibitor of active FXIIIa with (essentially) no influence on thrombin generation. It may thereby act as a potent protectant against thrombotic predispositions and complementary reducing endothelial inflammation, henceforth unwanted bleedings.

In the context of the present invention, the terms "polypeptide" and "peptide" may be understood interchangeably in the broadest sense as a compound mainly composed of amino acid moieties, preferably mainly composed of proteinogenic amino acid moieties, consecutively conjugated with another via amide bonds.

The person skilled in the art generally knows the proteinogenic amino acid moieties and its abbreviations in three-letter code and one-letter code. In addition, the polypeptide of the present invention may include one or more further non-proteinogenic amino acid moieties. For example, these may include one or more further amino acid moieties selected from the group consisting of citrulline (Cit), ornithine (Orn), and S-methylcysteine (Cys(Me)).

Optionally, ornithine may be used for replacing lysine and citrulline may be used for arginine. This may increase proteolytic stability and may thus increase half-life and may even allow new routes of administrations, including e.g. transdermal or oral administration. S-methylcysteine may be used as a replacement of cysteine to prevent that the respective residue participates in a disulfide bond, while at the same time mimicking a blocked thiol function in cysteine.

As used herein, the terms "amide bond" and "peptide bond" of the backbone structure may be understood

interchangeably as any -CO-NH-, -CO-NR$^x$- group, or a pharmaceutically acceptable derivative thereof, wherein R$^x$ and R$^y$ are each independently from another any organic moiety preferably comprising not more than 20 carbon atoms, more preferably a moiety selected from the group consisting of an optionally substituted $C_1$-$C_6$-alkyl, an optionally substituted $C_1$-$C_6$-alkylene-$C_3$-$C_{10}$-aryl, an optionally substituted $C_1$-$C_6$-alkylene-$C_3$-$C_{10}$-heteroaryl, an optionally substituted $C_1$-$C_6$-heteroalkyl, an optionally substituted $C_1$-$C_6$-heteroalkylene-$C_3$-$C_{10}$-aryl, and an optionally substituted $C_1$-$C_6$-heteroalkylene-$C_3$-$C_{10}$-heteroaryl, an optionally substituted $C_1$-$C_6$-alkenyl, an optionally substituted $C_1$-$C_6$-alkenylene-$C_3$-$C_{10}$-aryl, an optionally substituted $C_1$-$C_6$-alkenylene-$C_3$-$C_{10}$-heteroaryl, an optionally substituted $C_1$-$C_6$-heteroalkenyl, an optionally substituted $C_1$-$C_6$-heteroalkenylene-$C_3$-$C_{10}$-aryl, and an optionally substituted $C_1$-$C_6$-heteroalkenylene-$C_3$-$C_{10}$-heteroaryl. As used in this context, a $C_1$-$C_6$-alkyl, a $C_1$-$C_6$-heteroalkyl, a $C_1$-$C_6$-alkylene, and a $C_1$-$C_6$-heteroalkylene may each be linear or cyclic, unbranched or branched.

In a preferred embodiment, most of the amino acid moieties, in particular all of the amino acid moieties, of the polypeptide of the present invention are alpha amino acids, typically having the structure -NH-CR$^x$R$^y$-CO-, wherein the moieties R$^x$ and R$^y$ are defined as above, in particular wherein R$^x$ is hydrogen, i.e., having a structure NH-CHR$^y$-CO-.

As used herein, "hetero" may be understood in the broadest sense in that the respective moiety such as a side chain of an amino acid moiety contains one or more heteroatoms binding two or more carbon atoms, wherein the one or more heteroatoms may optionally be selected from the group consisting of nitrogen (N), oxygen (O), sulfur (S), selene (Se), a sulfate group, a sulfonate group, a phosphate group, a phosphonate group, a silicate group, a silane group, an ureate, an ester, a thioester, an amide, a guanidinium group, an imine, and a combination of two or more thereof. It will be understood that such atoms or functional groups will be each bound with the respective binding partners, including carbon atoms and hydrogen or other atoms, to fulfill its binding properties and that such atoms or functional groups may optionally also form a salt, in particular when binding or releasing one or more protons.

As used herein, the term "substituted" may be understood in the broadest sense in that one or more hydrogen atoms are replaced by one or more heteroatoms or functional groups. For example, a hydrogen atom may be replaced by an atom or functional group selected from the group consisting of deuterium, fluorine, a hydroxyl group, a thiol group, a sulfate group, a sulfonate group, a phosphate group, a phosphonate group, a silicate group, a silane group, an ureate, a carboxylic group, and a combination of two or more thereof.

As used in the context of the present invention, the term "pharmaceutically acceptable salt" may be understood in the broadest sense as generally understood in the art. A pharmaceutically acceptable salt may be any salt that may be administered to a patient without harming the patient.

For example, a pharmaceutically acceptable cation may be selected from the group consisting of one or more cations of sodium, potassium, magnesium, calcium, ammonium, and (preferably positively charged) amino acids. For example, a pharmaceutically acceptable anion may be selected from the group consisting of one or more anions of chloride, sulfate, phosphate, acetic acid, and (preferably negatively charged) amino acids.

The polypeptide of the present invention may also comprise one or more peptidomimetic moieties and may optionally be mainly or completely composed of peptidomimetic moieties and/or may optionally be a peptidomimetic structure as a whole. As used in the context of the present invention, the term "peptidomimetic" may be understood in the broadest sense as any mimic of a peptide that has similar properties like a peptide, but typically bears higher (biological) stability.

In a preferred embodiment, a peptidomimetic in the sense of the present invention is selected from the group consisting of beta amino acid moieties, N-acetylated amino acid moieties (e.g., N-methylated amino acid moieties) and peptoids (i.e., poly-N-substituted glycinyl moieties). The polypeptide may be partly or completely composed of peptidomimetic moieties. In a preferred embodiment, if the polypeptide is a peptidomimetic, all amino acid moieties of the polypeptide are amino acid analogues of one type (e.g. all are on beta amino acid moieties, all are N-acetylated amino acid moieties or all are N-substituted glycinyl moieties). Likewise, if the polypeptide is a D-peptide analogue, in a preferred embodiment, one or more amino acid moieties of the polypeptide may be D-amino acid moieties.

As used herein, the term "retro-inverso analogue" will be unambiguously understood by a person skilled in the art. A retro-inverso analogue may be understood as the respective sequence is reversed and/or D-amino acid moieties are used instead of L-amino acid moieties in different combinations.

The polypeptide may or may not comprise further chemical structures at one or both of its termini. In other words, the polypeptide may be an independent molecular structure (i.e., an unbound polypeptide) or a polypeptide conjugated to another molecular structure.

As indicated herein, the polypeptide of the present invention may or may not form a bridging moiety. In one embodiment the polypeptide comprises two intramolecular bridging moieties (e.g.one disulfide bond, one lactam bond or two lactam bonds).

In one embodiment, the polypeptide comprises a single (one) intramolecular bridging moiety (e.g. one lactam bond, or one disulfide bond). In one embodiment, the polypeptide does not comprise an intramolecular bridging moiety, i.e. is a linear polypeptide.

In a particularly preferred embodiment, the polypeptide comprises a single (one) lactam bond.

In preferred embodiments, two or none of $X^5$, $X^{17}$, $X^{31}$, and $X^{37}$ may be cysteine. In one embodiment, $X^{19}$ and $X^{25}$ are both Ala. In another embodiment, $X^{19}$ and $X^{25}$ are both Cys(Me). In another embodiment, $X^{19}$ is Ala and $X^{25}$ is Cys(Me). In another embodiment, $X^{19}$ is Cys(Me) and $X^{25}$ is Ala.

In a preferred embodiment, at least two of $X^5$, $X^{17}$, $X^{31}$, and $X^{37}$ are each independently selected from the group consisting of Cys(Me), Ser and Ala. In a preferred embodiment, $X^5$, $X^{17}$, $X^{31}$, and $X^{37}$ are each independently selected from the group consisting of Cys(Me), Ser and Ala.

In a preferred embodiment, $X^5$, $X^{17}$, $X^{31}$, and $X^{37}$ are each independently selected from the group consisting of Cys(Me), Ser and Ala. In a preferred embodiment, $X^5$, $X^{17}$, $X^{31}$, and $X^{37}$ are each Ser. In a preferred embodiment, $X^5$, $X^{17}$, $X^{31}$, and $X^{37}$ are each Ala. In a preferred embodiment, $X^5$, $X^{17}$, $X^{31}$, and $X^{37}$ are each Cys(Me).

In a preferred embodiment, one of $X^5$, $X^{17}$, $X^{31}$, and $X^{37}$ is Cys(Me) and the other ones are Ser or Ala. In a preferred embodiment, two of $X^5$, $X^{17}$, $X^{31}$, and $X^{37}$ are Cys(Me) and the other ones are each independently from each other Ser or Ala. In a preferred embodiment, three of $X^5$, $X^{17}$, $X^{31}$, and $X^{37}$ are Cys(Me) and the remaining moiety is Ser or Ala.

In a preferred embodiment, one of $X^5$, $X^{17}$, $X^{31}$, and $X^{37}$ is Ala and the other ones are Ser or Cys(Me). In a preferred embodiment, two of $X^5$, $X^{17}$, $X^{31}$, and $X^{37}$ are Ala and the other ones are each independently from each other Ser or Cys(Me). In a preferred embodiment, three of $X^5$, $X^{17}$, $X^{31}$, and $X^{37}$ are Ala and the remaining moiety is Ser or Cys(Me).

In a preferred embodiment, one of $X^5$, $X^{17}$, $X^{31}$, and $X^{37}$ is Ser and the other ones are Ala or Cys(Me). In a preferred embodiment, two of $X^5$, $X^{17}$, $X^{31}$, and $X^{37}$ are Serand the other ones are each independently from each other Alaor Cys(Me).

In a preferred embodiment, three of $X^5$, $X^{17}$, $X^{31}$, and $X^{37}$ are Ser and the remaining moiety is Ala or Cys(Me).

In another preferred embodiment, at least two, or all of $X^5$, $X^{17}$, $X^{31}$, and $X^{37}$ are pairwise selected from amino acid moieties able to from a lactam bond (either $X^5$-$X^{17}$, $X^5$-$X^{31}$, $X^5$-$X^{37}$, $X^{17}$-$X^{31}$, $X^{17}$-$X^{37}$ or $X^{31}$-$X^{37}$).

As used herein, a lactam bond may be understood in the broadest sense as an amide bond formed between two suitable amino acid side chains. Herein, a cyclic structure is formed. As used herein, an "amide bond" may be broadly defined as laid out above.

As used herein, an amino acid moiety forming a lactam bond with another one of the amino acid moieties selected from the group consisting of $X^5$, $X^{17}$, $X^{31}$, and $X^{37}$ may be any amino acid moiety that comprises a carboxyl group or an amino group in its side chain. Typically, a carboxyl group of a side chain of one amino acid moiety may react with an amino group of the side chain of another amino acid moiety.

In this context, an amino acid moiety having a carboxylic group ($-COOH$/ $-COO^-$) in its side chain may be any amino acid moiety having a carboxylic group in its side chain. It may be a naturally occurring amino acid moiety or an amino acid moiety of artificial origin. In a preferred embodiment, an amino acid moiety having a carboxylic group in its side chain may be selected from the group consisting of glutamic acid (Glu) and aspartic acid (Asp). In a preferred embodiment, an amino acid moiety having a carboxylic group in its side chain is Asp.

In this context, an amino acid moiety having an amino group ($-NH_2$/ $-NH_3^+$) in its side chain may be any amino acid moiety having an amino group in its side chain. It may be a naturally occurring amino acid moiety or an amino acid moiety of artificial origin. In a preferred embodiment, an amino acid moiety having an amino group in its side chain may be selected from the group consisting of lysine (Lys), ornithine (Orn), and 2,3-diaminopropionic acid (Dap). In a preferred embodiment, an amino acid moiety having an amino group in its side chain is Orn.

In a preferred embodiment, one or two lactam bonds may be formed by a combination of amino acid moieties selected from the group consisting of Asp/Lys, Asp/Orn, Asp/Dap, Glu/Lys, Glu/Orn, and Glu/Dap in any orientation.

A lactam bond may be prepared by any means. In a preferred embodiment, it is prepared by using orthogonal protecting groups at the side chains of an amino acid moiety having an amino group and/or an amino acid moiety having a carboxylic group. This allows the preparation of a specific lactam group.

In a preferred embodiment, the N-terminus of polypeptide strand is:

(i) acetylated;
(ii) an unbound primary amino group;
(iii) conjugated to a peptidic moiety, a non-peptidic moiety or a moiety comprising peptidic and non-peptidic structure elements, of a total molecular weight of not more than 500 Da,
(iv) conjugated to a peptidic polymeric structure, a non-peptidic polymeric structure or a polymeric structure comprising peptidic and non-peptidic structure elements, of a total molecular weight of more than 500 Da, preferably of 500 to 100,000 Da; or
(v) conjugated to solid support, optionally via a peptidic linker structure, a non-peptidic linker structure or a linker structure comprising peptidic and non-peptidic structure elements, preferably wherein the total linker structure has a molecular weight of 100 to 10,000 Da.

In a preferred embodiment, the C-terminus of polypeptide strand is:

(i) amidated,
(ii) an unbound carboxylic group;
(iii) conjugated to a peptidic moiety, a non-peptidic moiety or a moiety comprising peptidic and non-peptidic structure elements, of a total molecular weight of not more than 500 Da;
(iv) conjugated to a peptidic polymeric structure, a non-peptidic polymeric structure or a polymeric structure comprising peptidic and non-peptidic structure elements, of a total molecular weight of more than 500 Da, preferably of 500 to 100,000 Da; or
(v) conjugated to solid support, optionally via a peptidic linker structure, a non-peptidic linker structure or a linker structure comprising peptidic and non-peptidic structure elements, preferably wherein the total linker structure has a molecular weight of 100 to 10,000 Da.

As used throughout the present invention, a peptidic moiety may be comprised of one or more amino acid residues, which may be natural amino acids, non-natural amino acids or a combination thereof.

It will be understood that an amino acid residue is not necessarily be bound via the alpha amino group. For instance, a lysyl residue may also be bound via its epsilon amino group.

As used throughout the present invention, a non-peptidic moiety may be comprised of any chemical structure that is not an amino acid. For example, this may be a carboxylic acid, preferably of 1 to 20 carbon atoms in lengths, which may optionally be saturated or unsaturated, and may optionally comprise one or more further functional groups in addition to a carboxylic group. For example, this may be an amino group-containing moiety, preferably of 1 to 20 carbon atoms in lengths, which may optionally be saturated or unsaturated, and may optionally comprise one or more further functional groups in addition to an amino group. When conjugated to a hydrophobic structure, the polypeptide of the present invention may also be attached to or embedded in a micelle or liposome or microsome or nanoparticles or microparticles, SFD, HME (typically non-covalently). A non-peptidic moiety may optionally also be a dye (e.g., a fluorescence dye).

Optionally, the polypeptide of the present invention may be conjugated to another chemical moiety such as, e.g., a cell-penetrating peptide (e.g. polyarginine) for improving intracellular uptake and/or improving half-life and/or the plasma protein binding.

As used throughout the present invention, a peptidic polymeric structure may be any polypeptide structure. In one embodiment, a peptidic polymeric structure is a polypeptide of the present invention. In another embodiment, a peptidic polymeric structure is another polypeptide. In a preferred embodiment, a peptidic polymeric structure is a serum protein, in particular serum albumin.

As used throughout the present invention, a non-peptidic polymeric structure may be any structure that is not a polypeptide. In a preferred embodiment, a non-peptidic polymeric structure is polyethylene glycol (PEG).

As used throughout the present invention, a polymeric support may be any solid structure. In a preferred embodiment, a solid support is an endoprosthesis, in particular such as described below.

As used throughout the present invention, a linker structure is any bivalent structure that can link the polypeptide of the present invention with a biocompatible polymer. For instance, a linker structure may comprise polyethylene glycol (PEG).

Two or none of the amino acid moieties $X^5$, $X^{17}$, $X^{31}$, and $X^{37}$ may be a cysteine residue (Cys), which may optionally be or not be involved in a disulfide bond, in particular an intramolecular disulfide bond, i.e. forming a disulfide bond between amino acid moieties $X^5$ and $X^{37}$, $X^5$ and $X^{31}$, $X^{17}$ and $X^{37}$ or $X^{17}$ and $X^{31}$. In a preferred embodiment, a disulfide bond is formed between amino acid moieties $X^5$ and $X^{37}$. In another preferred embodiment, a disulfide bond is formed between amino acid moieties $X^{17}$ and $X^{31}$.

In a preferred embodiment, amino acid moieties $X^5$, $X^{17}$, $X^{31}$, and $X^{37}$ are each not Cys.

As indicated above, any of the amino acid moieties of the polypeptide of the present invention may optionally be replaced by its respective D-amino acid moiety.

In one embodiment, none of the amino acid moieties of the polypeptide is replaced by a D-amino acid moiety.

In another embodiment, one or more amino acid moieties of the polypeptide are replaced by the respective D-amino acid moieties. In a preferred embodiment, one or more amino acid moieties of the N-terminal part of the polypeptide ranging from amino acid moieties at positions 1 to 37 are replaced by the respective D-amino acid moieties.

In a preferred embodiment, at least one of the moieties selected from the group consisting of $Lys^6$, $Ile^{12}$, $Leu^{23}$ or $Ile^{34}$ is replaced by the respective D-amino acid residue. In a preferred embodiment, at least two of the moieties selected from the group consisting of $Lys^6$, $Ile^{12}$, $Leu^{23}$ or $Ile^{34}$ are each replaced by the respective D-amino acid residues. In a preferred embodiment, at least three of the moieties selected from the group consisting of $Lys^6$, $Ile^{12}$, $Leu^{23}$ or $Ile^{34}$ are each replaced by the respective D-amino acid residues. In a preferred embodiment, the moieties selected from the group consisting of $Ile^{12}$, $Leu^{23}$ or $Ile^{34}$ are each replaced by the respective D-amino acid residues.

In a preferred embodiment, $Lys^6$ is replaced by the respective D-amino acid residue. In a preferred embodiment, $Ile^{12}$ is replaced by the respective D-amino acid residue. In a preferred embodiment, $Leu^{23}$ is replaced by the respective D-amino acid residue. In a preferred embodiment, $Ile^{34}$ is replaced by the respective D-amino acid residue.

In a preferred embodiment, at $Lys^6$ and $Ile^{12}$ are replaced by the respective D-amino acid residue. In a preferred embodiment, at $Lys^6$ and $Leu^{23}$ are replaced by the respective D-amino acid residue. In a preferred embodiment, at $Lys^6$ and $Ile^{34}$ are replaced by the respective D-amino acid residue.

In a preferred embodiment, $Ile^{12}$ and $Leu^{23}$ are each replaced by the respective D-amino acid residue. In a preferred embodiment, $Ile^{12}$ and $Ile^{34}$ are each replaced by the respective D-amino acid residue. In a preferred embodiment, $Ile^{12}$ and $Ile^{34}$ are each replaced by the respective D-amino acid residue. In a preferred embodiment, $Leu^{23}$ and $Ile^{34}$ are each replaced by the respective D-amino acid residue.

In a preferred embodiment, $Lys^6$, $Ile^{12}$ and $Leu^{23}$ are each replaced by the respective D-amino acid residue. In a preferred embodiment, $Ile^{12}$, $Leu^{23}$ and $Ile^{34}$ are each replaced by the respective D-amino acid residue. In a preferred embodiment, $Lys^6$, $Leu^{23}$ and $Ile^{34}$ are each replaced by the respective D-amino acid residue. In a preferred embodiment, $Lys^6$, $Ile^{12}$ and $Ile^{34}$ are each replaced by the respective D-amino acid residue.

In a preferred embodiment, $Lys^6$, $Ile^{12}$, $Leu^{23}$ and $Ile^{34}$ are each replaced by the respective D-amino acid residue.

Optionally, one or more lysine moieties of the original tridegin sequence may be replaced by ornithine (Orn). Optionally, one or more arginine moieties of the original tridegin sequence may be replaced by citrulline (Cit).

In a preferred embodiment, one or more amino acid moiety positions $X^1$, $X^6$, $X^{45}$ and $X^{63}$ are each independently from each other selected from the group consisting of D-Lys and Orn and/or one or more amino acid moiety positions $X^{16}$, $X^{38}$, $X^{40}$, $X^{53}$, $X^{65}$ are each independently from each other selected from the group consisting of D-Arg and Cit.

In a preferred embodiment, amino acid moiety positions $X^1$, $X^6$, $X^{45}$ and $X^{63}$ are each independently from each other selected from the group consisting of D-Lys and Orn and amino acid moiety positions $X^{16}$, $X^{38}$, $X^{40}$, $X^{53}$, $X^{65}$ are each independently from each other selected from the group consisting of D-Arg and Cit.

In a preferred embodiment, one, two, three, or all of amino acid moiety positions $X^1$, $X^6$, $X^{45}$ and $X^{63}$ are each Orn.

In a preferred embodiment, amino acid moiety position $X^1$ is Orn. In a preferred embodiment, amino acid moiety position $X^6$ is Orn. In a preferred embodiment, amino acid moiety position $X^{45}$ is Orn. In a preferred embodiment, amino acid moiety position $X^{63}$ is Orn.

In a preferred embodiment, amino acid moiety positions $X^1$ and $X^6$ are each Orn. In a preferred embodiment, amino acid moiety positions $X^1$ and $X^{45}$ are each Orn. In a preferred embodiment, amino acid moiety positions $X^1$ and $X^{63}$ are each Orn. In a preferred embodiment, amino acid moiety positions $X^6$ and $X^{45}$ are each Orn. In a preferred embodiment, amino acid moiety positions $X^6$ and $X^{63}$ are each Orn. In a preferred embodiment, amino acid moiety positions $X^{45}$ and $X^{63}$ are each Orn.

In a preferred embodiment, amino acid moiety positions $X^1$, $X^6$ and $X^{45}$ are each Orn. In a preferred embodiment, amino acid moiety positions $X^6$, $X^{45}$ and $X^{63}$ are each Orn. In a preferred embodiment, amino acid moiety positions $X^1$, $X^6$ and $X^{63}$ are each Orn. In a preferred embodiment, amino acid moiety positions $X^1$, $X^{45}$ and $X^{63}$ are each Orn.

In a preferred embodiment, amino acid moiety positions $X^1$, $X^6$, $X^{45}$ and $X^{63}$ are each Orn.

In a preferred embodiment, one, two, three, four or all of amino acid moiety positions $X^{16}$, $X^{38}$, $X^{40}$, $X^{53}$ $X^{65}$ are each Cit.

In a preferred embodiment, amino acid moiety position $X^{16}$ is Cit. In a preferred embodiment, amino acid moiety position $X^{38}$ is Cit. In a preferred embodiment, amino acid moiety position $X^{40}$ is Cit. In a preferred embodiment, amino acid moiety position $X^{53}$ is Cit. In a preferred embodiment, amino acid moiety position $X^{65}$ is Cit.

In a preferred embodiment, amino acid moiety positions $X^{16}$ and $X^{38}$ are each Cit. In a preferred embodiment, amino acid moiety positions $X^{16}$ and $X^{40}$ are each Cit. In a preferred embodiment, amino acid moiety positions $X^{16}$ and $X^{53}$ are each Cit. In a preferred embodiment, amino acid moiety positions $X^{16}$ and $X^{65}$ are each Cit. In a preferred embodiment, amino acid moiety positions $X^{38}$ and $X^{40}$ are each Cit. In a preferred embodiment, amino acid moiety positions $X^{38}$ and $X^{53}$ are each Cit. In a preferred embodiment, amino acid moiety positions $X^{38}$ and $X^{65}$ are each Cit. In a preferred embodiment, amino acid moiety positions $X^{40}$ and $X^{53}$ are each Cit. In a preferred embodiment, amino acid moiety positions $X^{40}$ and $X^{65}$ are each Cit. In a preferred embodiment, amino acid moiety positions $X^{53}$ and $X^{65}$ are each Cit.

In a preferred embodiment, three of amino acid moiety positions $X^{16}$, $X^{38}$, $X^{40}$, $X^{53}$, $X^{65}$ are each Cit. In a preferred embodiment, four of amino acid moiety positions $X^{16}$, $X^{38}$, $X^{40}$, $X^{53}$, $X^{65}$ are each Cit. In a preferred embodiment, amino acid moiety positions $X^{16}$, $X^{38}$, $X^{40}$, $X^{53}$, $X^{65}$ are each Cit.

In a preferred embodiment, one or more amino acid moiety positions $X^1$, $X^6$, $X^{45}$ and $X^{63}$ are each Orn and/or one or more amino acid moiety positions $X^{16}$, $X^{38}$, $X^{40}$, $X^{53}$, $X^{65}$ are each Cit.

In a preferred embodiment, amino acid moiety positions $X^1$, $X^6$, $X^{45}$ and $X^{63}$ are each Orn and amino acid moiety positions $X^{16}$, $X^{38}$, $X^{40}$, $X^{53}$, $X^{65}$ are each Cit.

In a preferred embodiment, the polypeptide comprises at least one non-proteinogenic amino acid moiety, preferably selected from the group consisting of a D-amino acid moiety, Cit and Orn. In a preferred embodiment, the polypeptide comprises at least two non-proteinogenic amino acid moieties, preferably each independently selected from the group consisting of a D-amino acid moiety, Cit and Orn. In a preferred embodiment, the polypeptide comprises at least three non-proteinogenic amino acid moieties, preferably each independently selected from the group consisting of a D-amino acid moiety, Cit and Orn. In a preferred embodiment, the polypeptide comprises at least four non-proteinogenic

amino acid moieties, preferably each independently selected from the group consisting of a D-amino acid moiety, Cit and Orn. In a preferred embodiment, the polypeptide comprises at least five non-proteinogenic amino acid moieties, preferably each independently selected from the group consisting of a D-amino acid moiety, Cit and Orn.

In a preferred embodiment, the polypeptide comprises at least six non-proteinogenic amino acid moieties, preferably each independently selected from the group consisting of a D-amino acid moiety, Cit and Orn. In a preferred embodiment, the polypeptide comprises at least seven non-proteinogenic amino acid moieties, preferably each independently selected from the group consisting of a D-amino acid moiety, Cit and Orn. In a preferred embodiment, the polypeptide comprises at least eight non-proteinogenic amino acid moieties, preferably each independently selected from the group consisting of a D-amino acid moiety, Cit and Orn. In a preferred embodiment, the polypeptide comprises at least nine non-proteinogenic amino acid moieties, preferably each independently selected from the group consisting of a D-amino acid moiety, Cit and Orn.

In a preferred embodiment, the polypeptide comprises at least one non-proteinogenic amino acid moiety within the N-terminal amino acid moieties 1 to 37, preferably selected from the group consisting of a D-amino acid moiety, Cit and Orn. In a preferred embodiment, the polypeptide comprises at least two non-proteinogenic amino acid moieties within the N-terminal amino acid moieties, preferably each independently selected from the group consisting of a D-amino acid moiety, Cit and Orn. In a preferred embodiment, the polypeptide comprises at least three non-proteinogenic amino acid moieties within the N-terminal amino acid moieties, preferably each independently selected from the group consisting of a D-amino acid moiety, Cit and Orn. In a preferred embodiment, the polypeptide comprises at least four non-proteinogenic amino acid moieties within the N-terminal amino acid moieties, preferably each independently selected from the group consisting of a D-amino acid moiety, Cit and Orn. In a preferred embodiment, the polypeptide comprises at least five non-proteinogenic amino acid moieties within the N-terminal amino acid moieties, preferably each independently selected from the group consisting of a D-amino acid moiety, Cit and Orn. In a preferred embodiment, the polypeptide comprises at least six non-proteinogenic amino acid moieties within the N-terminal amino acid moieties, preferably each independently selected from the group consisting of a D-amino acid moiety, Cit and Orn.

In a preferred embodiment, the polypeptide comprises at least one non-proteinogenic amino acid moiety, selected from the group consisting of one or more respective D-amino acid moieties at one or more amino acid moiety positions Lys$^{6}$, Ile$^{12}$, Leu$^{23}$ and lie$^{34}$; Orn at one or more amino acid moiety positions X$^{1}$, X$^{6}$, X$^{45}$ and X$^{63}$; and Cit at one or more amino acid moiety positions X$^{16}$, X$^{38}$, X$^{40}$, X$^{53}$, X$^{65}$.

In a preferred embodiment, the polypeptide comprises at least two, at least three, at least four, at least five, at least six, at least eight, or at least nine non-proteinogenic amino acid moiety, selected from the group consisting of one or more respective D-amino acid moieties at one or more amino acid moiety positions Lys$^{6}$, Ile$^{12}$, Leu$^{23}$ and Ile$^{34}$;

Orn at one or more amino acid moiety positions X$^{1}$, X$^{6}$, X$^{45}$ and X$^{63}$; and
Cit at one or more amino acid moiety positions X$^{16}$, X$^{38}$, X$^{40}$, X$^{53}$, X$^{65}$.

In a preferred embodiment, the polypeptide comprises (or consists of) a polypeptide strand of SEQ ID NO: 1:

$$X^{1}LLPX^{5}X^{6}EWHQGIPNPX^{16}X^{17}WX^{19}GADLEX^{25}AQDQYX^{31}AFIPQX^{37}X^{38}PX^{40}SELIX^{45}PMDDIYQX^{53}PVEFPNLPLX^{63}PX^{65}E,$$

wherein amino acid moieties X$^{1}$, X$^{6}$, X$^{45}$ and X$^{63}$ are each independently selected from the group consisting of Lys, D-Lys, and Orn;
wherein amino acid moieties X$^{16}$, X$^{38}$, X$^{40}$, X$^{53}$, X$^{65}$ are each independently selected from the group consisting of Arg, D-Arg, and Cit;
wherein amino acid moieties X$^{5}$, X$^{17}$, X$^{31}$, and X$^{37}$ are each independently selected from the group consisting of Ser, Ala, Cys(Me), an amino acid moiety forming a lactam bond with another one of the amino acid moieties selected from the group consisting of X$^{5}$, X$^{17}$, X$^{31}$, and X$^{37}$, and Cys optionally forming a disulfide bond with another Cys at an amino acid moiety selected from the group consisting of X$^{5}$, X$^{17}$, X$^{31}$, and X$^{37}$;
wherein not more than two of X$^{5}$, X$^{17}$, X$^{31}$, and X$^{37}$ are Cys;
wherein amino acid moieties X$^{19}$ and X$^{25}$ are each independently selected from the group consisting of Cys(Me), Ser, and Ala,
or a pharmaceutically acceptable salt of the sequence SEQ ID NO: 1,
wherein optionally one or more of the L-amino acid moieties may be replaced by the respective one or more D-amino acids,
or a retro-inverso analogue of the sequence of SEQ ID NO: 1,
or a peptidomimetic analogue of the sequence of SEQ ID NO: 1.

In a preferred embodiment, the polypeptide comprises (or consists of) a polypeptide strand selected from the group consisting of KLLPC*k*EWHQG*i*PNPRCWSGAD*l*ESAQDQYCAF*i*PQCRPRSELIKPMDDIYQRPV EFPNLPLKPRE (SEQ ID NO: 2), wherein $k^6$ is a D-lysyl moiety, $l^{23}$ is a D-leucine and $i^{12}$ and $i^{34}$ are D-isoleucine moieties,

$X^1$LLPS$X^6$EWHQGIPNP$X^{16}$SWSGADLESAQDQYSAFIPQS$X^{38}$P$X^{40}$SELI$X^{45}$PMDD IYQ$X^{53}$PVEFPNLPL$X^{63}$P$X^{65}$E (SEQ ID NO: 3),

wherein $X^1$, $X^6$, $X^{45}$ and $X^{63}$ are each Orn; and wherein $X^{16}$, $X^{38}$, $X^{40}$, $X^{53}$, $X^{65}$ are each Cit,

KLLPAKEWHQGIPNPRAWSGADLEAAQDQYAAFIPQARPRSELIKPMDDIYQRP VEFPNLPLKPRE (SEQ ID NO: 4),

and

$X^1$LLPA$X^6$EWHQGIPNP$X^{16}$AWSGADLEAAQDQYAAFIPQA$X^{38}$P$X^{40}$SELI$X^{45}$PMDD IYQ$X^{53}$PVEFPNLPL$X^{63}$P$X^{65}$E (SEQ ID NO: 5),

wherein $X^1$, $X^6$, $X^{45}$ and $X^{63}$ are each Orn; and wherein $X^{16}$, $X^{38}$, $X^{40}$, $X^{53}$, $X^{65}$ are each Cit,
wherein optionally one or more of the L-amino acid moieties may be replaced by the respective one or more D-amino acids,
or a retro-inverso analogue of any one of the sequences,
or a peptidomimetic analogue of any one of the sequences,
or a pharmaceutically acceptable salt of any one of the sequences.

In a preferred embodiment, each polypeptide comprising a polypeptide strand of any one of SEQ ID NOs: 1 to 5 is acetylated at the N-terminus (i.e., the N-terminal alpha nitrogen, Ac-NH-) and amidated at the C-terminus (i.e., the C-terminal carboxyl group, $-CO-NH_2$).

In one embodiment, one or more cysteine moieties may optionally be each substituted by Cys(Me). In one embodiment, each cysteine moiety may optionally be substituted by Cys(Me).

In one embodiment, one or more serine moieties may optionally be each substituted by Cys(Me), in particular of one or more serine residues that replace cysteine residues e.g., in SEQ ID NO: 3). In one embodiment, each serine moiety may optionally be substituted by Cys(Me), in particular of one or more serine residues that replace cysteine residues e.g., in SEQ ID NO: 3).

In one embodiment, one or more alanine moieties may optionally be each substituted by Cys(Me), in particular of one or more alanine moieties that replace cysteine residues e.g., in SEQ ID NOs: 4 and/or 5). In one embodiment, each alanine moiety may optionally be substituted by Cys(Me), in particular of one or more alanine residues that replace cysteine residues e.g., in SEQ ID NOs: 4 and/or 5).

As indicated above, the polypeptide may or may not comprise a bridging moiety. In other words, the polypeptide may be cyclic (i.e., with one or two intramolecular bridges) or may be linear (i.e., without an intramolecular bridge). As noted above, in some preferred embodiments, the polypeptide may optionally comprise one or two disulfide bonds.

In a preferred embodiment, the polypeptide does not comprise a disulfide bond. This may help to avoid disulfide shuffling and/or disulfide modification. This may reduce the number of structural isomers. In this context, it may be noted that a lactam bond may be optionally used instead that may avoid shuffling.

In a preferred embodiment, the polypeptide does not comprise an intramolecular bridge. In this case, it is a linear polypeptide. In another preferred embodiment, the polypeptide comprises one or two lactam bonds (e.g., between one or more Asp or Glu moieties and an equivalent number of Lys or Orn or Dap moieties). Such a lactam bond may optionally replace a disulfide bond. It may optionally be at the corresponding positions of the sequence.

In a preferred embodiment a lactam bond is formed between amino acid moieties $X^5$ and $X^{37}$ and/or a lactam bond is formed between amino acid moieties $X^{17}$ and $X^{31}$ or between amino acid moieties $X^5$ and $X^{17}$ and between amino acid moieties $X^{31}$ and $X^{37}$, or between amino acid moieties $X^5$ and $X^{31}$ and between amino acid moieties $X^{17}$ and $X^{37}$.

In another preferred embodiment, a lactam bond may be formed between amino acid moieties $X^5$ and $X^{37}$. In a preferred embodiment, $X^5$ is Asp or Glu and $X^{37}$ is Lys or Orn or Dap and a lactam bond is formed between these groups. In another preferred embodiment, $X^5$ is Lys or Orn or Dap and $X^{37}$ is Asp or Glu and a lactam bond is formed between these

groups. In a particularly preferred embodiment, $X^5$ is Asp and $X^{37}$ is Orn and a lactam bond is formed between these groups. In another particularly preferred embodiment, $X^5$ is Orn and $X^{37}$ is Asp and a lactam bond is formed between these groups.

In another preferred embodiment, a lactam bond may be formed between amino acid moieties $X^{17}$ and $X^{31}$. In another preferred embodiment, $X^{17}$ is Asp or Glu and $X^{31}$ is Lys, Orn or Dap and a lactam bond is formed between these groups. In another preferred embodiment, $X^{17}$ is Lys, Orn or Dap and $X^{31}$ is Asp or Glu and a lactam bond is formed between these groups. In another preferred embodiment, $X^{17}$ is Asp and $X^{31}$ is Orn and a lactam bond is formed between these groups. In another preferred embodiment, $X^{17}$ is Orn and $X^{31}$ is Asp and a lactam bond is formed between these groups.

In one embodiment, a lactam bond may be formed between amino acid moieties $X^5$ and $X^{37}$ and a further lactam bond may be formed between amino acid moieties $X^{17}$ and $X^{31}$. In a more preferred embodiment, a single lactam bond may be formed between amino acid moieties $X^5$ and $X^{37}$ or between amino acid moieties $X^{17}$ and X31.

The polypeptide of the present invention may be obtained by any means known for this purpose in the art. In a preferred embodiment, the polypeptide of the present invention is obtained by solid phase peptide synthesis (SPPS) such as of Fmoc- or Boc-based SPPS. Alternatively, the polypeptide of the present invention may also be obtained by liquid phase peptide synthesis (LPPS) or, in the case of consisting of L-amino acid moieties, by means of biotechnology means such as heterologous expression in a genetically modified organism excluding human bodies such as, e.g., bacteria (e.g., *E. coli*), fungi (e.g., yeast), mammalian cells or mammalians excluding humans, insect cells or insects, plant cells or plants, etc. In some host cells, one or more posttranslational modifications may occur. Accordingly, genetic manipulation of a host organism with the sequence comprising the polypeptide of the present invention may be used.

In SPPS (or also LPPS), the synthesis is typically based on the stepwise coupling of amino acid moieties bearing protected side chains (orthogonal protecting groups). Typically, during synthesis, the peptide strand grows from the C-terminus to the N-terminus. However, there are alternative methods wherein the peptide strand grows from the N-terminus to the C-terminus. Nowadays, the most common methods are based on at least two or more different types of protecting groups that are cleavable under at least two or more different conditions, such as, e.g., the fluorenyl-9-methoxycarbonyl/tert-butyl- (Fmoc/tBu) protecting group scheme (Sheppard Tactics) or the tert-butoxycarbonyl/benzyl- (Boc/Bzl) protecting group scheme (Merrifield Tactics).

A protecting group may be any protecting group known in the art such as, e.g., an amino-protecting group of the urethane type (e.g., benzyloxycarbonyl (Z), 4-methoxybenzyloxycarbonyl (Z(OMe)), 2-nitrobenzyloxycarbonyl (Z(2-$NO_2$)), 4-nitrobenzyloxycarbonyl (Z($NO_2$)), chlorobenzyloxycarbonyl (Z(Cl), Z(2-Cl), Z(3-Cl), Z(2,4-Cl)), 3,5-dimethoxybenzyloxycarbonyl (Z(3,5-OMe), alpha,alpha-dimethyl-3,5-dimethoxybenzyloxycarbonyl (Ddz), 6-nitroveratryloxycarbonyl (Nvoc), 4-(phenyldiazenyl)-benzyloxycarbonyl (Pz), 2-(biphenyl-4-yl)-2-propoxycarbonyl (Bpoc), isonicotinyloxycarbonyl (iNoc), tert-butoxycarbonyl (Boc), 2-cyano-*tert*-butoxycarbonyl (Cyoc), 2,2,2-trichloro-tert-butoxycarbonyl (Tcboc), adamantyl-1-oxycarbonyl (Adoc), 1-(1-adamantyl)-1-methoxycarbonyl (Adpoc), fluorenyl-9-methoxycarbonyl (Fmoc), (2-nirofluoren-9-yl)methoxycarbonyl (Fmoc($NO_2$)), 2-(4-toluenesulfonyl)-ethoxycarbonyl (Tsoc), methylsulfonylethoxycarbonyl (Msc), 2-(4-nitrophenylsulfonyl)ethoxycarbonyl (Nsc), 2-(tert-butylsulfonyl)-2-propenyloxycarbonyl (Bspoc), 1,1-dioxobenzo[b]-thien-2-ylmethoxycarbonyl (Bsmoc), 2-/methylsulfonyl)-3-phenyl-2-propenyloxycarbonyl (Mspoc), allyloxycarbonyl (Aloc), 2-(trimethylsilyl)-ethoxycarbonyl (Teoc), triisopropylsilylethoxycarbonyl (Tipseoc), piperidinyloxycarbonyl (Pipoc), cyclopententyloxycarbonyl (Poc)), a carboxy-protecting group of the ester type (e.g., methyl (Me), ethyl (Et), benzyl (Bzl), 4-nitrobenzyl (Nbz), 4-methoxybenzyl (Mob), 2,4-di methoxybenzyl (2,4-Dmb), o-chlorotrityl (Trt(2-Cl), pyrimidyl-4-methyl(4-picolyl (Pic), phenacyl (Pac), 4-methoxyphenacyl (Pac(OMe), diphenylmethyl (Dpm), tert-butyl (tBu), cyclohexyl (Cy), 1-adamantyl (1-Ada), 2-adamantyl (2-Ada), dicyclopropylmethyl (Dcpm), 9-phenylfluoren-9-yl (Pf), 9-fluorenylmethyl (Fm), 2-trimethylsilylethyl (TMSE), 2-phenyl-trimethyl-silyl (PTMSE), allay (Al), 4-{N-[1-(4,4-dimethyl-2,6-dioxocyclohexanylidene)-3-methylbutyl]-amino}benzyl (Dmab)), a thiol-protecting group (e.g., benzyl (Bzl), 4-methylbenzyl (Bzl(4-Me)), 4-methoxybenzyl (Mob), 2,4,6-trimethoxybenzyl (Tmb), diphenylmethyl (Dpm), trityl (Trt), tert-butyl (tBu), acetamidomethyl (Acm), trimethylacetamidomethyl (Tacm), 9-fluorophenylmethyl (Fm), tert-butylsulfanyl (StBu), 3-nitro-2-pyridylsulfanyl (Npys), 9H-xanthen-9-yl (Xan)), an imidazole protecting group (e.g., benzyl (Bzl), 2,4-dinitrophenyl (Dnp), benzyloxycarbonyl (Bom), adamantly-1-oxycarbonyl (Adoc), triphenylmethyl (Trt), diphenylmethyl (Dpm), pyridyldiphenylmethyl (Pdpm), 4-toluenesulfonyl (Tosyl, Tos), 4-methoxybenzenesulfonyl (Mbs), tert-butoxymethyl (Bum), allyl (Al), allyloxymethyl (Alom)), an a hydroxyl-protecting group (e.g., benzyl (Bzl), 2,6-dichlorobenzyl (Dcb), diphenylmethyl (Dbm), cyclohexyl (Cy), 2-bromobenzyloxycarbonyl (Z(2-Br)), tert-butyl (tBu), 1-benzyloxycarbonyl-amino-2,2,2-trifluoroethyl (Zte), methylthiomethyl (Mtm), allyl (Al), or a guanidinium protecting group (e.g., 2,2,4,6,7-pentamethyldihydrobenzofuran-5-sulfonyl (Pbf), tert-butoxycarbonyl (Boc), 2,2,5,7,8-pentamethylchroman-6-sulfonyl (Pmc)).

It will be known that in SPPS, the peptide is typically bound to a solid support during synthesis. In the context of peptide synthesis, a solid support may be understood interchangeably in the broadest sense as any solid matrix known for peptide synthesis in the art. Typically, the solid support used during peptide synthesis is a polymeric bead. The solid support may be, but may not be limited to, chloromethyl resin (Merrifield resin), 4-benzyloxybenzyl alcohol resin (Wang

resin), (2,4-dimethoxy)benzhydrylamine resin (Rink amide resin), 2,4-dialkoxybenzyl resin (super acid-sensitive resin, SASRIN®), 2-chlorotrityl resin, alpha-chlorotritylchloride resin (Barlos resin), benzhydrylamine resin (BHA resin), chloromethyl resin, hydroxymethylbenzoic acid resin (HMBA resin), 4-hydroxymethyl-3-methoxyphenoxybutyric acid resin (HMPB resin), hydroxycrotonoyl aminomethyl resin (HYCRAM resin), MBHA resin, oxime resin, Sieber resin, Ramage-amide resin and/or a resin with special cleavable linkers (e.g., photolabile linkers or safety-catch linkers).

Alternatively or additionally, the polypeptide may be also provided by conjugating two or more peptide strand(s) with another by any conjugation method known in the art such as, e.g., Native Chemical Ligation (NCL), Click Chemistry, maleimide-thiol conjugation, enzymatic conjugation, biochemical protein ligation and/or soluble handling conjugation.

Additionally, the polypeptide may be optionally purified by any means known in the art, such as, e.g., one or more chromatographic methods (e.g. high-performance liquid chromatography (HPLC), preferably reversed phase HPLC (RP-HPLC), ultrahigh-performance liquid chromatography (UPLC or UHPLC), fast protein liquid chromatography (FPLC), etc.), one or more filtration methods (e.g., one or more diafiltration methods, one or more dead-end filtration methods, one or more crossflow filtration methods), one or more electrophoretic methods, one or more precipitation-based methods, one or more dialysis methods, one or more other concentration methods, one or more precipitation steps (e.g., salting in, salting out, contacting with an anti-solvent, etc.), one or more crystallization steps, one or more freeze-drying steps (also: lyophilization), or a combination of two or more thereof.

Additionally, the polypeptide may optionally be oxidized to form one intramolecular disulfide bond (also: cystine bond, including the structural pattern -S-S-). The optional formation of a disulfide bond may depend on the primary structure of the polypeptide. It may also depend of the secondary structure of the polypeptide.

Optionally, oxidation may be accomplished by storage of the polypeptide in an oxygen-containing environment (e.g., in a solution or at dry state), by purging oxygen in a solution containing the polypeptide or pharmaceutically acceptable salt thereof, and/or by adding one or more oxidizing agents to a solution containing the polypeptide or pharmaceutically acceptable salt thereof.

When expressed in an organism, i.e., prepared by biotechnological means, it will be understood that a polypeptide in the sense of the present invention may or may not be subjected to one or more posttranslational modifications. The one or more optional posttranslational modifications typically depend on the organism in which the polypeptide of interest is expressed (in case of being prepared by biotechnological means).

The termini of the polypeptide may optionally be capped by any means known in the art, such as, e.g., amidation, acetylation, methylation, and/or acylation. Posttranslational modifications are well-known in the art and may be but may not be limited to lipidation, phosphorylation, sulfatation, glycosylation, truncation, oxidation, reduction, decarboxylation, acetylation, amidation, deamidation, disulfide bond formation, hydroxylation, amino acid addition, cofactor addition (e.g., biotinylation, heme addition, eicosanoid addition, steroid addition) and complexation of metal ions, non-metal ions, peptides or small molecules and addition of iron-sulphide clusters. Moreover, optionally, co-factors, in particular cyclic guanidinium monophosphate (cGMP), but optionally also such as, e.g., ATP, ADP, NAD+, NADH+H+, NADP+, NADPH+H+, metal ions, anions, lipids, etc. may be bound to the polypeptide, irrespective on the biological influence of these co-factors. It will be understood that such polypeptide may also contain one or more non-natural amino acid moiety/moieties and/or one or more posttranscriptional modifications.

The polypeptide of the present invention may be stored at any condition suitable for such purpose. For example, it may be dried or freeze-dried. For example, it may be stored in a temperature range of -100 to 30°C, or -80 to 30°C, or -25 to 25°C, or -25 to 0°C, or 0 to 25°C, or 2 to 22°C, or 5 to 15°C, or 10 to 25°C, or 15 to 25°C, or 18 to 22°C. For example, it may be stored at ambient temperature (e.g., 15 to 30°C, preferably 18 to 25°C), in a fridge (e.g., 2 to 20°C), a freezer (e.g., -25 to -5°C), a deep freezer (e.g., -80 to -60°C), or in a liquid gas (e.g., -150 to -90°C).

As indicated above, in some embodiments, it is beneficial when the polypeptide of the present invention is pharmaceutically administrable to a patient.

The polypeptide of the present invention may be administered to a patient in any form. Accordingly, in a preferred embodiment, the polypeptide of the present invention forms part of a pharmaceutical composition.

Thus, a further aspect of the present invention is related to a pharmaceutical composition comprising:

(A) the polypeptide of the present invention; and
(B) at least one pharmaceutically acceptable carrier.

As used herein, the terms "pharmaceutical composition" and "pharmaceutical formulation" may be understood interchangeably as any composition that may be used in a pharmaceutical context such as for treating or preventing a patient.

As used herein, the terms "pharmaceutically acceptable carrier", "pharmaceutically acceptable excipient", "carrier" and "excipient" may be understood interchangeably in the broadest sense as any substance that may support the pharmacological acceptance of the polypeptide of the present invention. Such pharmaceutical composition may be ready to use and may preferably be a liquid formulation, in particular an injection portion. The storage form may also be

liquid, but may also be a dried form (e.g. a powder such as a powder comprising dried or freeze-dried polypeptide of the present invention) or may be a paste or syrup or the like. Optionally, a dried form, paste or syrup may be dissolved or emulsified prior to being administered to the patient.

A pharmaceutically acceptable carrier may exemplarily be selected from the list consisting of an aqueous buffer, saline, water, dimethyl sulfoxide (DMSO), ethanol, vegetable oil, paraffin oil or combinations of two or more thereof. Furthermore, the pharmaceutically acceptable carrier may optionally contain one or more detergent(s), one or more foaming agent(s) (e.g., sodium lauryl sulfate (SLS), sodium dodecyl sulfate (SDS)), one or more coloring agent(s) (e.g., food coloring), one or more vitamin(s), one or more salt(s) (e.g., sodium, potassium, calcium, zinc salts), one or more humectant(s) (e.g., sorbitol, glycerol, mannitol, propylenglycol, polydextrose), one or more enzyme(s), one or more preserving agent(s) (e.g., benzoic acid, methylparaben, one or more antioxidant(s), one or more herbal and plant extract(s), one or more stabilizing agent(s), one or more chelating agents (e.g., ethylenediaminetetraacetic acid (EDTA), and/or one or more uptake mediator(s) (e.g., polyethylene imine (PEI), a cell-penetrating peptide (CPP), a protein transduction domain (PTD), an antimicrobial peptide, etc.).

The present invention also relates to a dosage unit of the pharmaceutical composition usable in the context of the treatment or prevention of the present invention. Exemplarily, the present invention may refer to a single dose container or to a multiple dosage form.

Preferably, a pharmaceutical composition is prepared for final administration to enable routes of administration, which circumvent the first pass effect. More preferably, the pharmaceutical composition is prepared to be suitable for administration by injection into the patient (e.g., suitable for administration routes selected from the group consisting of intravenous (i.v.), intraarterial (i.a.), intraperitoneal (i.p.), intramuscular (i.m.), and subcutaneous (s.c.) injection). Alternatively or additionally, the pharmaceutical composition may also be suitable for other routes of administration such as, e.g., nasal or transdermal administration.

The polypeptide of the present invention may optionally also be included in or attached to a liposome and/or a micelle.

The polypeptide and the pharmaceutical composition may be used for any purpose.

As indicated above, the polypeptide may be used to treat and/or prevent a thrombotic event.

Accordingly, a further aspect of the present invention relates to the polypeptide of the present invention or a pharmaceutical composition of the present invention for use in a method for treating or preventing a thrombotic event in a patient.

It will be understood that the definitions and preferred embodiments laid out in the context of the polypeptide and the pharmaceutical composition as laid out above *mutatis mutandis* apply to the pharmaceutical use thereof.

In other words, the present invention relates to a method for treating or preventing a thrombotic event in a patient, wherein the patient is administered with a sufficient amount of the polypeptide of the present invention or a pharmaceutical composition of the present invention.

As used in the context of the present invention, the term "patient" may be understood in the broadest sense as any living being, which is preferably an animal, more preferably a mammal including human, in particular a human being. A non-human mammal as used herein may be any non-human mammal, preferably a domestic or agriculturally used animal such as, e.g., a horse, a dog, a cat, a bovine, a goat, a sheep, a donkey, or a camel.

The polypeptide of the present invention or a pharmaceutical composition of the present invention may be administered to the patient by any means. In a preferred embodiment, it is administered to the patient by a route of administration, which circumvents the first pass effect. In a preferred embodiment, the polypeptide or a pharmaceutical composition is administered to the patient by injection, in particular injection selected from the group consisting of intravenous (i.v.), intraarterial (i.a.), intraperitoneal (i.p.), intramuscular (i.m.), and subcutaneous (s.c.) injection. In an alternative preferred embodiment, the polypeptide or a pharmaceutical composition is administered by nasal or transdermal administration. Alternatively, also other routes of administration such as, e.g., transdermal or oral administration may be used.

The term "thrombotic event" may be understood in the broadest sense as generally understood in the art. Typically, a thrombotic event includes the formation of one or more blood clots. The thrombotic event may be a vascular thrombotic event (VTE).

In a preferred embodiment, the patient is at risk of developing or is suffering from thrombosis or embolization of the large blood vessels. In another preferred embodiment, the patient is at risk of developing or is suffering from microthrombi. In another preferred embodiment, the patient is at risk of developing or is suffering from thrombosis or embolization of the large blood vessels and microthrombi. As used herein, large blood vessels may have an inner diameter of the lumen of at least 2 mm and microthrombi are thrombi occurring in blood vessels having an inner diameter of the lumen of below 2 mm.

In a preferred embodiment, the patient is suffering from or is at risk of developing or a pathological state associated with a thrombotic event selected from the group consisting of stenosis of one or more veins, venules, arteria, arterioles, and/or capillaries.

A stenosis of one or more veins and/or venules may be any type of at least partly occlusion of one or more veins and/or venules hampering blood flow in one or more veins and/or venules.

In a preferred embodiment, stenosis of veins and/or venules is selected from the group consisting of deep vein thrombosis, pelvic vein thrombosis, and retinal vein occlusion.

A stenosis of one or more arteries and/or arterioles may be any type of at least partly occlusion of one or more arteries and/or arterioles. In a preferred embodiment, stenosis of one or more arteries and/or arterioles may be atherosclerosis or stenosis of arteries.

A stenosis of one or more capillaries may be any type of at least partly occlusion of one or more capillaries. In a preferred embodiment, stenosis of capillaries may be an angiopathy coincidence with thrombotic events in capillary flow path, in particular diabetic angiopathy.

In a preferred embodiment, the patient is suffering from one or more ischemic regions that lead to or are at risk of leading to necrosis of at least a part of a tissue without administration of the polypeptide or pharmaceutical salt thereof of the present invention to said patient.

In a preferred embodiment, the patient is suffering from or is at risk of developing or a pathological state associated with a thrombotic event selected from the group consisting of stroke, myocardial infarction, embolism, disseminated intravascular coagulation (DIC), thrombotic thrombocytopenic purpura (TTP), thrombophlebitis, sepsis, or a combination of two or more thereof.

Embolism may be embolism of any tissue. In a preferred embodiment, embolism is pulmonary embolism.

Infarction may be infarction of any organ. In a preferred embodiment, infarction is myocardial infarction.

The administration of the polypeptide of the present invention may enhance reperfusion and reduce the recurrence of one or more thrombotic events in postoperative settings. Optionally, the administration of the polypeptide of the present invention may reduce FXIII-mediated, optionally local, inflammation such as, e.g., in case of arthropathies. In one embodiment, locally reversing thrombotic events may be destined by FXIIIa mediated reactions such as alpha2-macroglobulin placement within the growing thrombotic event (e.g., blood clot), reduced platelet adhesion via reduced alpha2-antiplasmin incorporation. Children with severe thrombotic events (e.g., blood clots) may benefit from shorter treatments (tailored treatments for young children). As this peptide inhibitor may have a shorter half-life than many synthetic agents, it may significantly reduce recurrent thrombotic event risk as well as (subsequent) bleeding risk. The half-life may be adapted by routes of administration as well as structural modifications.

In a preferred embodiment, the patient is subjected to a surgery. Surgery may be any type of surgery. In a preferred embodiment, surgery is bypass surgery. The polypeptide may be administered to the patient prior to a surgery, may be administered to the patient subsequent to receiving a surgery, or may be administered to the patient prior to and subsequent to receiving a surgery.

In a preferred embodiment, the patient is receiving an endoprosthesis. An endoprosthesis may be any type of an endoprosthesis. In a preferred embodiment, an endoprosthesis is selected from the group consisting of a blood vessel endoprosthesis (e.g., a stent, artificial vessel, etc.), an artificial joint (e.g., an artificial hip joint, an artificial knee joint, etc.), a cardiac pacemaker, an artificial bowel outlet, and a cosmetic implant.

The target FXIIIa may be associated with the regulation of Insulin resistance and adipogenesis. Stent failures among T2D patients often involve endothelial dysfunction, and platelet hyperactivity, which in turn is again expected to raise local FXIIIa. Administration of FXIIIa inhibitor to the stent (coated with the polypeptide) may improve biofunctionalization by improving stent endothelium interactions.

In a preferred embodiment, the patient is receiving an organ transplant and/or a tissue transplant. As used herein, an organ transplant may be understood in the broadest sense. For instance, an organ transplant may be a transplant of an inner organ (e.g., a kidney, a liver, a heart, a stomach, a part of a gut, or a blood vessel, etc.). As used herein, a tissue transplant may be understood in the broadest sense. For instance, a tissue transplant may be a transplant of tissue of an inner organ (e.g., of liver, of lung, of gut), or retina, or of skin.

As noted above, the polypeptide and the pharmaceutical composition may be used for any purpose. The polypeptide may optionally be used for a coating such as, e.g., for an endoprosthesis.

Accordingly, a yet further aspect of the present invention relates to an endoprosthesis or an enzyme-linked immunosorbent assay (ELISA) plate coated with the polypeptide or pharmaceutically acceptable salt thereof.

It will be understood that the definitions and preferred embodiments laid out in the context of the polypeptide or pharmaceutically acceptable salt thereof, the pharmaceutical composition and use thereof as laid out above *mutatis mutandis* apply to the endoprosthesis coated with the polypeptide or pharmaceutically acceptable salt thereof.

As used herein, the term "endoprosthesis" may be understood in the broadest sense as any artificial implant that remains in the patient's body. Typically, an endoprosthesis is of a solid material and may thus be considered as a solid support of the polypeptide or pharmaceutically acceptable salt thereof. The endoprosthesis may be any endoprosthesis. In a preferred embodiment, the endoprosthesis is selected from the group consisting of a blood vessel endoprosthesis (e.g., a stent, artificial vessel, etc.), an artificial joint (e.g., an artificial hip joint, an artificial knee joint, etc.), a cardiac pacemaker, an artificial bowel outlet, and a cosmetic implant (e.g., a breast implant, a chin implant, a buttock implant, etc.).

Coating may be performed by any means. In a preferred embodiment, coating is a covalent binding of the polypeptide to the solid endoprosthesis. Such covalent binding may be directly or may be via a linker structure. A linker structure may be any linker structure. In a preferred embodiment, a linker structure is a peptidic linker structure, a non-peptidic linker structure or a linker structure comprising peptidic and non-peptidic structure elements, preferably wherein the total linker structure has a molecular weight of 100 to 10,000 Da. For instance, a linker may have a molecular weight of 100 to 1,000 Da, or 200 to 2,000 Da, or 500 to 5,000 Da, or 1,000 to 10,000 Da.

Alternatively or additionally, coating may involve complex formation between the polypeptide and the solid endoprosthesis. This may be achieved by any type of complex formation such as, e.g., by a chelate complex (e.g., a histidine tag bound to a nickel ion). A complex may also be formed with a linker structure as described before.

In another preferred embodiment, coating is a non-covalent coating.

This may be performed by any means such as, e.g., by soaking and/or spraying of the endoprosthesis with a solution of the polypeptide or pharmaceutically acceptable salt thereof.

It will be understood that the polypeptide may also be used for any non-therapeutic purpose.

In one aspect, the polypeptide is used in assay development such as, e.g. in an enzyme-linked immunosorbent assay (ELISA) involving FXIIIa or an enzyme kinetic test based on FXIIIa. Herein, the polypeptide may specifically bind to FXIIIa and may replace antibodies. The polypeptide may optionally be labelled (e.g., fluorescently, by an enzyme, by colloidal gold, by a binding group, etc.). This may enable fluorescence-based localization/visualization. Also, cellular assays may be possible that involve binding of FXIIIa. The functionality and biochemical processes of formation of thrombotic events may be further revealed. The polypeptide may also serve as a pharmacological tool for *in vitro* and ex *vivo* testing and chromogenic as well as fluorogenic FXIIIa activity assays.

The following examples, claims and figures are intended to provide illustrative embodiments of the present invention.

**Brief description of the Figures**

**Figure 1** shows an overview of tridegin derivatives. These may be tridegin derivatives having two disulfide bonds between $Cys^{5}/Cys^{17}$ and $Cys^{31}/Cys^{37}$ (Fig. 1A, also designated as "isomer A" herein) and tridegin derivatives having two other disulfide bonds between $Cys^{5}/Cys^{37}$ and $Cys^{5}/Cys^{17}$ (Fig. 1A, also designated as "isomer B" herein) and tridegin derivatives having two other disulfide bonds between (Fig. 1A, also designated as "isomer C" herein). Furthermore, an NMR-based structural characterization of the double-bridged isomer B (Fig. 1B) and a superimposition of the NMR-based structural characterization with the previously used exclusively computer-based model of isomer B (Fig. 1C) are depicted.

**Figure 2** shows a functional *in vitro* analysis of tridegin derivatives. (A) Curve for fluorogenic enzyme activity assay of polypeptide isomer B (n=3). (B) $IC_{50}$ values derived from the fluorogenic enzyme activity assay for the investigated polypeptides isomer B (SEQ ID NO: 6) (1), SEQ ID NO: 2 (2), All-Ser (SEQ ID NO: 7) (3), SEQ ID NO: 3 (4), SEQ ID NO: 4 (5), SEQ ID NO: 5 (6), SEQ ID NO: 8 (7), SEQ ID NO: 9 (8), SEQ ID NO: 10 (9), and SEQ ID NO: 11 (10). (C) Relative inhibitory effects at 1.1 $\mu$M polypeptide concentration obtained by the Berichrom® assay. Inhibitory effects were normalized to 100% for polypeptide isomer B (SEQ ID NO: 6). (D) FXIIIa inhibition upon addition of several polypeptides isomer B (SEQ ID NO: 6) (1), SEQ ID NO: 2 (2), and All-Ser (SEQ ID NO: 7) (3) at varying concentrations in the Berichrom® assay (n = 4). Activity is correlated to a standard human plasma with a reference interval of 70 to 140%. Consequently, data points below 15% were considered inactive. (E-F) Whole blood clot contraction assay of polypeptide isomer B (SEQ ID NO: 6) and polypeptide All-Ser (SEQ ID NO: 7) performed in human whole blood (n = 1) (E) and polypeptide All-Ser (SEQ ID NO: 7) performed in whole blood from mice (n = 1) (F). x represents inhibition by irreversible FXIIIa inhibitor T101 (Zedira GmbH).

**Figure 3** shows an *in vivo* analysis of the effect of polypeptide isomer B (SEQ ID NO: 6) on FXIIIa activity in mice.

Examples

Preparation of polypeptides (tridegin derivatives)

Different tridegin derivatives were prepared by standard solid phase peptide synthesis (SPPS).

*Experimental section*

Materials. All solvents, reagents, and buffer chemicals were of analytical grade if not stated otherwise. Further details about the chemicals are given below.

**Synthesis of peptides and purification.** *General procedure for SPPS.* Peptide synthesis of all peptides was carried out by automated solid phase peptide synthesis (SPPS) using a standard Fmoc (N-(9-fluorenyl)methoxy-carbonyl) protocol on an EPS 221 synthesizer (Intavis Bioanalytical Instruments AG, Cologne, Germany). A Rink amide MBHA resin (0.56 mmol/g) was utilized. The coupling reagent 2-(1H-benzotriazol-1-yl)-1,1,3,3-tetramethyluronium hexafluorophosphate (HBTU) and the base N-methylmorpholine were applied to perform coupling of the Fmoc-amino acid derivatives. Fmoc deprotection was carried out with 20% piperidine in dimethylformamide. Introduction of non-proteinogenic amino acids ornithine, citrulline and the D-amino acid derivatives of Lys, Ile and Leu was achieved by using Fmoc-L-Orn(Boc)-OH, Fmoc-L-Cit-OH, Fmoc-D-Lys(Boc)-OH, Fmoc-D-Ile-OH, and Fmoc-D-Leu-OH as derivatives. For the selective formation of disulfide bridges Fmoc-L-Cys(Trt)-OH and Fmoc-L-Cys(Acm)-OH at the respective positions have been applied as outlined earlier.

Peptide cleavage and side-chain deprotection (except for acetamidomethylated cysteine) were accomplished by addition of a cocktail of 1.0 ml reagent K (75 mg phenol, 25 μl 1,2-ethandithiol, 50 μl thioanisol, 50 μl water in 0.95 ml TFA) per 100 mg resin on ice and subsequent stirring for 3 h at room temperature. The cleavage mixture was filtered and precipitated in ice-cold diethyl ether. Peptide pellets were washed for three times with diethyl ether and re-dissolved in 80% tert-butanol for freeze-drying.

*Oxidation of linear precursor peptides.* Crude linear precursor peptides containing cysteines, i.e., peptides 1 and 2 were oxidized as described earlier with slight modifications as follows. Peptides (0.025 - 0.050 mM, 1 equiv.) in 50% acetic acid were mixed with 1.1 equiv. iodine (0.1 M in methanol) under argon for 1 h to complete the first oxidation. Subsequently, 8.9 equiv. iodine (0.1 M in methanol) were added to the same reaction mixture and left stirring for 4 h. The solution was diluted with the same volume of water and iodine was extracted 3 times with ethyl acetate. The aqueous phase was freeze-dried and stored at -20 °C.

*General procedure for purification of peptides.* Crude (oxidized) peptides were purified by semi-preparative RP-HPLC using a Shimdazu LC-8A system (Duisburg, Germany) or a JASCO PV-987 instrument (Gross-Umstadt, Germany) equipped with a Knauer Eurospher 100 column (C18, 250 × 32 mm, 5.0 μm particle size, 100 Å pore size). The separation was performed in a gradient elution system of eluent A (0.1% TFA in water) and eluent B (acetonitrile:/water 9/1, 0.1 % TFA). By continuously increasing the concentration of eluent B by 50 % within 120 min at a flow rate of 10 ml/min elution of the peptides was achieved and detected at 220 nm. The applied gradients for each peptide are described in Table 1 below.

Collected fractions were combined, evaporated from acetonitrile, freeze-dried, and stored at -20 °C. Peptide purity was confirmed by analytical HPLC on a Shimadzu LC-20AD system equipped with a Vydac 218TP column (C18, 250 × 4.6 mm, 5.0 μm particle size, 300 Å pore size). Gradient elution was carried out using acetonitrile containing 0.1 % TFA (eluent B) and 0.1 % TFA in water (eluent A) at a flow rate of 1.0 mL/min. The detection of the peptides was at $\lambda$ = 220 nm. Retention times and methods are given in Table 1 below.

All peptides were obtained in purities >95 %.

*Peptides analytical characterization.* Analytical HPLC (as aforementioned, or performed on a Shimadzu Prominence-i LC-2030 system equipped with a Vydac 208TP column (C8, 250 × 4.6 mm, 5.0 μm particle size, 300 Å pore size) in gradient elution mode using acetonitrile containing 0.1 % TFA (eluent B) and 0.1 % TFA in water (eluent A) at a flow rate of 1.0 mL/min), mass spectrometry, and thin-layer chromatography (TLC) were applied to verify peptide identities. Peptide molar masses were analyzed by matrix-assisted laser desorption-ionization/time-of-flight (matrix-assisted laser desorption/ ionization (MALDI), MALDI-TOF) on an ultrafleXtreme instrument. Peptide contents were determined by RP-HPLC using the above-mentioned LC-20AD system based on peak area comparison of previously determined peptide samples.

The tridegin derivative isomer B (SEQ ID NO: 6) containing disulfide bridges $C^5$-$C^{37}$ and $C^{17}$-$C^{31}$ was selected as a starting point for further analysis and optimization to evaluate its applicability for future drug development. In one tridegin derivative, all cysteine moieties were replaced by serine moieties (All-Ser, SEQ ID NO: 7). In another tridegin derivative, all cysteine moieties were replaced by alanine moieties (SEQ ID NO: 4). Replacements of amino acids in positions 19 and 25 were analyzed to identify feasibility and cost savings for the synthesis of such analogues (SEQ ID NO: 8) and to investigate the impact of the polarity of these amino acids using alternatives with similarity to a disulfide bridge between $C^{19}$ and $C^{25}$, i.e. S-methylcysteine (SEQ ID NO: 11).

At the same time, introduction of non-proteinogenic amino acids, such as D-amino acids, or replacements of amino acids by non-proteinogenic amino acids, such as ornithine for lysine and citrulline for arginine, were tested to reduce accessibility of these peptides for peptidases and proteases in the plasma or organism, respectively, depending on the route of administration. As a consequence, further modifications of polypeptides All-Ser (SEQ ID NO: 7) or SEQ ID NO: 4 by substituting all lysine moieties and arginine moieties for ornithine moieties and citrulline moieties, respectively, have been carried out and yielded polypeptides SEQ ID NO: 3 and SEQ ID NO: 5. In another derivative of SEQ ID NO: 7, K' and

$L^{34}$ were replaced for the corresponding D-amino acids (SEQ ID NO: 9, SEQ ID NO: 10). Indeed, synthesis yielded for the linear peptides without disulfide bridges significantly increased since oxidation of cysteines as an additional synthesis step was not required anymore.

The aforementioned peptides are as follows (wherein the experimentally used polypeptides amidated at the C-terminus (i.e., the C-terminal carboxyl group, $-CO-NH_2$):

Polypeptide No. 1, Polypeptide isomer B:

KLLPCKEWHQGIPNPRCWSGADLESAQDQYCAFIPQCRPRSELIKPMDDIYQRPVEFPNLPLKPRE (SEQ ID NO: 6),

wherein preferably disulfide bonds form between cysteine residues $Cys^{5}$ and $Cys^{37}$ as well as between $Cys^{17}$ and $Cys^{31}$.

Polypeptide No. 2:

KLLPC*k*EWHQG*i*PNPRCWSGAD*l*ESAQDQYCAF*i*PQCRPRSELIKPMDDIYQRPVEFPNLPLKPRE (SEQ ID NO: 2),

wherein $k^{6}$ is a D-lysyl moiety, $l^{23}$ is a D-leucine and $i^{12}$ and $i^{34}$ is a D-isoleucine moiety, and wherein preferably disulfide bonds form between cysteine residues $Cys^{5}$ and $Cys^{37}$ as well as between $Cys^{17}$ and $Cys^{31}$.

Polypeptide No. 3, Polypeptide All-Ser:

KLLPSKEWHQGIPNPRSWSGADLESAQDQYSAFIPQSRPRSELIKPMDDIYQRPVEFPNLPLKPRE (SEQ ID NO: 7).

Polypeptide No. 4:
O*rn*LLPS*Orn*EWHQGIPNP*Cit*SWSGAOLESAQOQYSAFIPQS*Cit*P*Cit*SELI*Orn*PM DDIYQ*Cit*PVEFPNLPL*Orn*P-*Cit*E, which can also be depicted as:

$X^{1}$LLPS$X^{6}$EWHQGIPNP$X^{16}$SWSGADLESAQDQYSAFIPQS$X^{38}$P$X^{40}$SELI$X^{45}$PMDDIYQ$X^{53}$PVEFPNLPL$X^{63}$P$X^{65}$E (SEQ ID NO: 3),

wherein $X^{1}$, $X^{6}$, $X^{45}$ and $X^{63}$ are each Orn; and wherein $X^{16}$, $X^{38}$, $X^{40}$, $X^{53}$, $X^{65}$ are each Cit.

Polypeptide No. 5:

KLLPAKEWHQGIPNPRAWAGADLEAAQDQYAAFIPQARPRSELIKPMDDIYQRPVEFPNLPLKPRE (SEQ ID NO: 4).

Polypeptide No. 6:
*Orn*LLPA*Orn*EWHQGIPNP*Cit*AWAGADLEAAQDQYAAFIPQA*Cit*P*Cit*SELI*Orn*PM DDIYQ*Cit*PVEFPNLPL*Orn*P-*Cit*E, which can also be depicted as

$X^{1}$LLPA$X^{6}$EWHQGIPNP$X^{16}$AWAGADLEAAQDQYAAFIPQA$X^{38}$P$X^{40}$SELI$X^{45}$PMDDIYQ$X^{53}$PVEFPNLPL$X^{63}$P$X^{65}$E (SEQ ID NO: 5),

wherein $X^{1}$, $X^{6}$, $X^{45}$ and $X^{63}$ are each Orn; and wherein $X^{16}$, $X^{38}$, $X^{40}$, $X^{53}$, $X^{65}$ are each Cit.

Polypeptide No. 7:

KLLPSKEWHQGIPNPRSWAGADLEAAQDQYSAFIPQSRPRSELIKPMDDIYQRPVEFPNLPLKPRE (SEQ ID NO: 8).

Polypeptide No. 8:

*k*LLPSKEWHQGIPNPRSWAGADLEAAQDQYSAFIPQSRPRSELIKPMDDIYQRPVEFPNLPLKPRE (SEQ ID NO: 9),

wherein $k^1$ is a D-lysyl moiety.

Polypeptide No. 9:

KLLPSKEWHQGIPNPRSWAGAD*l*EAAQDQYSAFIPQSRPRSELIKPMDDIYQRPVEFPNLPLKPRE (SEQ ID NO: 10),

wherein $l^{23}$ is a D-leucine moiety.

Polypeptide No. 10:

KLLPSKEWHQGIPNPRSW$X^{19}$GADLE$X^{25}$AQDQYSAFIPQSRPRSELIKPMDDIYQRPVEFPNLPLKPRE (SEQ ID NO: 11),

wherein $X^{19}$ and $X^{25}$ are each Cys(Me).

As experimentally used, the polypeptides were synthesized as C-terminal amides. Changes in cysteine positions compared to isomer B (SEQ ID NO: 6) are underlined above. Changes for other amino acids are given in italics in the respective sequences, e.g. *k* = D-Lys, *i* = D-Ile, etc.

Initially, a careful analysis of potential cleavage sites for peptidases and proteases was performed applying primarily the MEROPS database and yielded 51 potential cleavage sites for proteases and peptidases. This is depicted for polypeptide 2 (SEQ ID NO: 2), wherein, in the first line, the cleavage sites are depicted in bold letters, in the second line the solvent-accessible surface area is depicted in bold letters, in the third row, the accessible cleavage sites are depicted in bold letters, the fourth row the interaction sites (no change allowed) are depicted in bold letters, and in the last line, the accessible cleavage sites without interaction are depicted in bold letters (in SEQ ID NO: 6):

**K**LL**P**C**KEW**HQ**GIP**N**PR**C**WSGADLESA**Q**D**Q**Y**C**AFIP**QC**RPRSELIKP**M**DDIYQRPVEFPNLPLK**PR**E**

**KLL**PC**KEWHQ**G**I**PNPRCWSGAD**LE**SA**QDQ**YCA**FI**PQC**R**PRSE**LIKPM**D**DIYQR**PV**E**F**P**N**LP**L**KPRE**

**K**LLPC**KEW**HQG**I**PNPRCWSGAD**LE**SAQ**D**QYCA**FI**PQC**R**PRSE**LIKP**MD**DIYQRP**V**E**F**P**N**LP**L**K**PR**E**

**K**LLPCKE**WHQG**I**PN**PRC**W**SG**A**DLESA**QD**QYC**AF**IPQCRPRSELIKP**MDD**IYQ**RPVE**F**PNL**P**LK**P**RE**

KLLPC**KE**WHQG**I**PNPRCWSGAD**LE**SAQDQYCAF**I**PQC**R**PRSE**LIKP**MDD**IYQ**RPVEFPNL**P**LKPRE

In parallel, the solvent-accessible surface area of polypeptide isomer B (SEQ ID NO: 6) was examined considering the earlier reported simulations of this peptide. This allowed narrowing down the number of accessible cleavage sites to a total of 27 amino acids. Exclusion of relevant interaction sites with FXIIIa was implemented as a third criterion to decide for potential amino acids for replacement by e.g. D-amino acids in polypeptide isomer B (SEQ ID NO: 6) without affecting its inhibitory potential and reduced the number of relevant positions to 15. Considering that the main impact on the inhibitory potential of tridegin is inferred by the C-terminal (amino acids 38-66), further exchanges in this region in the beginning have been excluded, which enabled to limit our selection to six potential amino acid positions for replacement. In combination with computational studies, revealing which amino acids are of interest for the interaction of the N-terminal fragment with FXIIIa, we decided first to substitute the amino acid moieties $Lys^6$, $Ile^{12}$, $Leu^{23}$ and $Ile^{34}$ by

their corresponding D-amino acid. Mutations of $Glu^{7}$ and $Glu^{24}$ were avoided as glutamic acid cleavage sites appear with only very minor frequency in a physiological context. This led us to the design of polypeptide 2 (SEQ ID NO: 2). Analysis of the formed disulfide connectivities by the applied selective protecting group strategy was performed as earlier and confirmed the expected bonds between $Cys^{5}$-$Cys^{37}$ and $Cys^{17}$-$Cys^{31}$.

**Table 1.** Analytical characterization of the peptides.

| Polypeptide No.* | $t_R$ (min) | [M+H]+ (calc.)[a] | $[M+H]^+$ (meas.)[a] | $R_f1$ | $R_f2$ |
|---|---|---|---|---|---|
| **1** | 19.1[b] | 7741.8 | 7742.3 | 0.25[d] | 0.20[e] |
| **2** | 19.7[b] | 7741.8 | 7741.6 | 0.31[d] | 0.25[e] |
| **3** | Analytical studies were performed before | | | | |
| **4** | 20.0[b] | 7631.0 | 7630.8 | 0.64[f] | 0.15[e] |
| **5** | 20.3[b] | 7586.0 | 7586.0 | 0.94[g] | 0.04[e] |
| **6** | 21.8[b] | 7535.0 | 7535.2 | 0.64[f] | 0.11[e] |
| **7** | 18.6[b] / 38.5[c] | 7649.0 | 7649.6 | - | - |
| **8** | 18.7[b] / 38.6[c] | 7649.0 | 7649.8 | - | - |
| **9** | 18.1[b] / 38.0[c] | 7649.0 | 7649.9 | - | - |
| **10** | 19.0[b] / 38.9[c] | 7740.9 | 7741.5 | - | - |

*as indicated above.
[a]monoisotopic mass.
[b]20-60% in 40 min measured on a Shimadzu LC-20A system equipped with a C18 Vydac 218TP column.
[c]0-60% in 60 min measured on a Shimadzu Prominence-I LC20-30 system equipped with a C8 Vydac 208TP column.
[d]*tert*-butanol/acetic acid/ethyl acetate/water (1/1/1/1).
[e]pyridine/ethyl acetate/acetic acid/water (5/5/3/1).
[f]acetonitrile/water (1/1). [g]acetonitrile/water/25% ammonia (25/27/10).

**Functional studies.** *Enzyme activity* assay. Enzyme activity was measured using a fluorogenic enzyme activity assay. The measurements were carried out with the substrate H-Tyr(3-$NO_2$)-Glu(NH-(CH2)$_4$-NH-Abz)-Val-Lys-Val-Ile-$NH_2$ as described previously. $IC_{50}$ plots were fitted in GraphPad Prism 9.3 (Synergy Software, Reading, PA, USA) by nonlinear regression using the equation $y = v_{max}/(1 + (x/x_0)s)$ with y = response, $v_{max}$ = maximum velocity, x = inhibitor concentration, $x_0$ = relative $IC_{50}$, and s = slope factor. Results were subsequently illustrated in GraphPad Prism 9.3. $IC_{50}$ values are shown as the means ± standard deviation. *Berichrome*® assay *for FXIIIa activity.* For the assay, an inhibitor stock solution (11 mM in $H_2O$) was diluted with water to 5500, 1100, 550, and 110 µM. Each concentration was diluted 1:10 with normal pooled plasma (NPP). 15 µl of each dilution was added to a mixture of 75 µl activator reagent and 75 µl detection reagent (Siemens, Berichrom® Assay Kit). As a positive control, human plasma was mixed 10:1 with $H_2O$ and measured analogously. As negative control, FXIII-deficient plasma was used instead of human plasma. For detection, the released ammonia was used, which was converted into glutamate in a glutamate dehydrogenase side reaction. The decrease in NADH was determined photometrically at 340 nm. The measurements were carried out in triplicates on two different days. Data were evaluated applying GraphPad Prism 9.3.

*Whole blood and plasma preparation.* Phlebotomy was performed on consenting healthy donors in accordance with the Declaration of Helsinki and the University of North Carolina Institutional Review Board. Blood was collected by venipuncture using a 21G butterfly needle (Becton, Dickenson and Company, Franklin Lakes, NJ) into 0.105 M sodium citrate, pH 5.5 (10% v/v, final concentration). Normal pooled plasma (NPP) was obtained from four healthy donors.

For each donor, the first 5 ml were discarded. Platelet-poor plasma was prepared by sequential centrifugation (150 g for 25 min, then 20 000 g for 20 min), pooled, aliquoted, flash frozen with liquid nitrogen, and stored at -80 °C.

*Whole blood clot contraction assay*. Clotting was triggered in recalcified (10 mM, final concentration) whole blood via the addition of a tissue factor (Innovin diluted 1:12 000, 1 pM, final concentration). Final reaction volumes were 200 µl (85% whole blood; 10% tridegin variant, T101, or HBS; 2.5% Innovin; and 2.5% calcium chloride). Clot contraction proceeded at 37 °C for 120 min in siliconized multiwell plates. Contracted clots were removed and weighed. Analysis of clot weight was performed in GraphPad v 7.02 (Synergy Software, Reading, PA, USA) using the equation $y = bottom + (top - bottom)/(1 + (x/IC_{50}))$ with y = response and x = inhibitor concentration. $IC_{50}$ values are shown as the means ± standard deviation. Statistical analysis was performed using an unpaired t-test (Holm-Sidak method) by means of GraphPad v 7.04 (Synergy Software, Reading, PA, USA). P values less than 0.01 were considered statistically significant.

***Pharmacokinetics.*** *Aqueous solubility.* Peptides were dissolved in 0.07 mM phosphate buffer (pH 7.4, 6.2 or 5.0) at 100 μM and incubated for 18 h at 25 °C. After incubation samples were centrifuged at 14000 rpm for 15 min and supernatants were again centrifuged for 15 min. No pellet formation was observed at any step for the investigated peptides. Supernatants were finally analyzed by analytical HPLC as described above applying a gradient of 20%-60% eluent B in 40 min. Peak areas were quantified and compared to a standard curve of polypeptide TS2 that was prepared in advance.

*Measurement of* $LogD_{7.4}$. $LogD_{7.4}$ was measured by adapting the shake flask procedure from OECD19 at room temperature in n-octanol (> 99 %) and 20 mM phosphate-buffer pH 7.4. The peptides (100 μg/ml) were dissolved in a solution with equal amounts of n-octanol and phosphate buffer, shaken for 1 h and measured for the amount of peptide in each phase with RP-HPLC. The $LogD_{7.4}$ values were estimated using the relation $\log([\text{peptide}]_{\text{octanol}}/[\text{peptide}]_{\text{buffer}})$.

*Plasma protein binding.* For the PPB studies, plasma (total volume 200 μl) was spiked with the peptides (10 μM) and incubated for 5 min for PPB equilibrium. The peptide containing plasma probes were transferred into 500 μl 30 kDa MWCO filter devices (Amicon Ultra, Merck, Darmstadt) and filled up with 300 μl PBS buffer.

The devices were centrifuged at 14,000 rcf for 15 min and subsequently washed with 500 μl of PBS buffer. This procedure was repeated once. After the elution of the filter concentrate, the slurry was exactly reconstituted with PBS buffer to a final volume of 200 μl. The PPB samples as well as the reference (10 μM of the peptide in 200 μl PBS) were diluted 1:100 with 0.2 % formic acid and finally measured via LC-MS.

*Plasma stability.* Compounds were incubated for 3 h in 1 ml human plasma at room temperature (10 μM and 100 μM). Reaction samples were taken at defined times and measured by selected reaction monitoring.

*Human liver microsomal stability.* Compounds were incubated for 1 h at room temperature at a concentration of 10 μM. Reaction samples were taken at defined times and measured by selected reaction monitoring.

*Preparation of cell cultures.* Human hepatocellular carcinoma (HepG2) cells were cultured in Dulbecco's modified Eagle's medium (DMEM, Gibco, Austria) supplemented with 10% fetal bovine serum (FBS; Gibco, Austria), penicillin (100 U/ml) and streptomycin (0.1 mg/ml) solution (Gibco, USA). All cells were cultured under a humidified $CO_2$ (5.0 %) atmosphere at 37°C and passaged by trypsinization when reached approximately 80% confluence. For experiments, cells in exponential phase of growth (at a density of 5,000 cells/well) were seeded into 96-well flat-bottom plates after treatment with trypsin-EDTA (Greiner, Germany) solution at a final volume of 100 μl/well. Cells were incubated overnight before treatment with test substances.

*Cell viability* assay. The cytotoxicity of the above-references six polypeptides (1.0 mM stock solutions in $PBS_{7.4}$) was evaluated in HepG2 cells by a colorimetric assay using 3-(4,5-dimethylthiazol-2-yl)-2,5-diphenyltetrazolium bromide (MTT). Cells were seeded in 96-well culture plates with a density of $2.5 \times 10^4$ cells per well. Following adherence, the cells were treated with the polypeptides at different concentrations (0.1, 1.0, 5.0, 10, 25, 50 and 100 μM) and further incubated for 24 h at 37°C. After incubation period, MTT solution (0.5 mg/ml) was added and cells were incubated for further 90 min. Then, the medium was removed and the plates were placed in a plate shaker at room temperature until complete dissolution of purple formazans. The quantification of formazans produced from the biological sample after reduction of MTT was monitored using a microplate ELISA reader VarioscanTM LUX (Thermo Fisher Scientific Inc., USA) at a wavelength of 540 nm with a reference wavelength of 630 nm. The cytotoxicity of the test peptides determined by MTT assay was expressed as percentage cell viability according to the following equation:

$$\%\text{Cell viability} = (A_{\text{sample}} - A_{\text{blank}})/(A_{\text{control}} - A_{\text{blank}}) \times 100$$

In this equation, $A_{\text{sample}}$, $A_{\text{blank}}$ and $A_{\text{control}}$ are the measured absorption of the respective test sample, blank solution, and control sample. The results were expressed as the mean % of the untreated controls ± SD from three independent experiments (n = 3).

**Computational Studies.** *MD simulations.* Molecular dynamics (MD) simulations in this study were carried out using the Gromacs 2018 package and the YASARA platform. In general, NMR studies may be conducted as described in the art (Schmitz et al., International Journal of Molecular Sciences, 2021, 22:880). The first model of the 100-member NMR ensemble of the full-length model of polypeptide TS2 was used as the starting structure of the 300-ns long simulation. The polypeptide was placed in the center of a cubic simulation cell, with the edges of the cube separated by at least 12 Å from all atoms of the peptide. The polypeptide was solvated using the TIP3P water model with additional Na+ and Cl- counter ions added to achieve a physiological salt concentration of 0.9%, while maintaining a zero net charge on the entire system. The solvated system was subjected to 5000 steps of steepest-descents energy minimization with the Amber-ff14sb force field, which was used to describe atomic motions for all parts of the simulation.

The energy-minimized system was first subjected to a temperature equilibration in the NVT (constant number of atoms, volume, temperature) ensemble for 2 ns, with temperature maintained by the velocity-rescaled variant of the Berendsen thermostat at 300 K. Subsequently a 2 ns simulation to equilibrate the pressure of the system at 1 atm was conducted in the NPT (constant number of atoms, pressure, temperature) ensemble aided by the Parrinello-Rahman

barostat. During both the temperature and pressure equilibration runs, all heavy atoms were position-restrained by the LINCS algorithm. The production run was conducted for 300 ns using a 2 fs timestep. Long-range interactions were cutoff at 10 Å, and the electrostatics were described by a particle-mesh (Ewald method). Periodic boundary conditions were employed in the simulations, the effects of which were adjusted prior to conducting analyses on the trajectory.

Trajectory analysis, namely the backbone root mean square deviation (RMSD) computation was conducted in VMD 1.9.3., which was also used in the creation of molecular graphics. Snapshots were written at a 100 ps interval to disc, resulting in a total of 3000 snapshots collected for analysis, from this simulation.

*Molecular docking studies.* The full-length model of polypeptide isomer B (SEQ ID NO: 6)2 was used for docking onto the modified and simulation-equilibrated crystal structure of FXIIIa originally derived from the source PDB ID: 4KTY. The PDB structure used as a receptor for docking was a simulation-averaged structure of the non-proteolytically activated FXIIIa, which had been modified, i.e., missing regions/loops filled, all heteroatoms/water molecules removed, and subjected to classical molecular dynamic simulation.

The details of this simulation have been reported earlier. The full-length model obtained as the final snapshot of the 300 ns MD simulation was used as the ligand for the docking simulation. Docking was performed on the Hdock server (http://hdock.phys.hust.edu.cn/) in a blind fashion and under the default conditions of the server. The Hdock server is based on a hybrid algorithm of template-based modeling and ab initio free docking and is currently ranked favorably amongst the topmost automated docking servers in the recent CASP (critical assessment of protein structure prediction) competitions. Only the top-ten docking poses generated by the server were closely inspected.

The docking pose that strongly agreed with the experimental data was subjected to a docking refinement protocol on the Haddock 2.2 webserver (https://milou.science.uu.nl/services/HADDOCK2.2/haddockserver-refinement.html) that allows for flexible refinement in explicit solvent of the docked complex. The model with the best Haddock score from the output clusters generated out of the refinement protocol was finally inspected in terms of interatomic interactions on the protein interaction calculator webserver (http://pic.mbu.iisc.ernet.in/). Similarly docking was also performed with the mutant structures (polypeptides isomer B (SEQ ID NO: 6) and 2 (SEQ ID NO: 2)) of the well-defined N-terminal fragment as the ligand and the above-described FXIIIa structure as the receptor.

**Functional studies of tridegin analogs**

As earlier reported, polypeptides 3 (SEQ ID NO: 7) and 5 (SEQ ID NO: 4) were used as controls and for reasons of comparison. In previous studies, inhibition of the target protein FXIIIa by tridegin was extensively investigated by a fluorogenic enzyme activity assay:

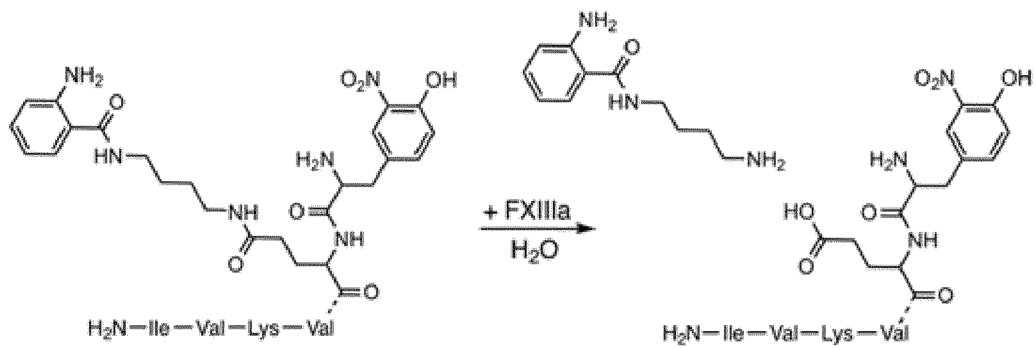


This assay is based on the isopeptidase activity of FXIIIa and releases a fluorophore upon isopeptide bond cleavage within the substrate used. The cleavage indicates full functional enzyme activity, which is changed in the presence of activity-modulating compounds and therefore can be determined accordingly.

The impact of the peptides investigated, herein on FXIIIa activity, was monitored using this fluorometric assay and the substrate H-Tyr(3-$NO_2$)-Glu(Dab-2-Abz)-Val-Lys-Val-Ile-$NH_2$. Linear progress curves for the above-mentioned polypeptides were recorded and indicated stable inhibition of FXIIIa as described earlier. The $Cys^{19}$-$Cys^{25}$-deficient tridegin derivatives (e.g., polypeptide isomer B (SEQ ID NO: 6)) displayed significant inhibition of FXIIIa with an $IC_{50}$ value of 1.02 ± 0.06 μM (n = 3) in a comparable way as was already described (Figs. 3A, 3B). Interestingly, the polypeptides 3 (SEQ ID NO: 7), 5 (SEQ ID NO: 4), 7 (SEQ ID NO: 8), and 10 (SEQ ID NO: 11), all of them lacking cysteines, revealed slightly reduced $IC_{50}$ values of 1.51 ± 0.11 μM (n = 3), 1.53 ± 0.11 μM (n = 3), 1.66 ± 0.09 μM (n = 3), and 1.76 ± 0.10 μM (n = 3), respectively. Linear polypeptides 8 (SEQ ID NO: 9, 2.06 ± 0.09 μM, n = 3) and 9 (SEQ ID NO: 10, 1.33 ± 0.08 μM, n = 3) with one D-amino acid provided results in a similar range. Polypeptide 2 (SEQ ID NO: 2) containing even four D-amino acids also maintained a significant inhibitory potential towards FXIIIa with an $IC_{50}$ of 1.93 ± 0.11 μM (n = 3). Although several amino acids have been exchanged in polypeptides 3 (SEQ ID NO: 7) and 5 (SEQ ID NO: 4), there was still some

inhibitory potency maintained for both peptides (4.76 ± 0.37 μM and 5.02 ± 0.34 μM, respectively).

Thus, all peptides investigated in this study, some representing proteolytically stabilized tridegin derivatives, were still potential reversibly binding inhibitors and none lost its activity completely despite the extensive number of amino acid changes or modifications that had been introduced in the sequence.

For validation of the results of the fluorogenic assay, the Berichrom® assay for determining the FXIIIa activity was applied for the most potent polypeptides isomer B (SEQ ID NO: 6), 2 (SEQ ID NO: 2), and 3 (SEQ ID NO: 7) ($IC_{50}$ < 2 μM). The polypeptides 5-10 (SEQ ID NO: 4 and 8-11) were excluded due to their very close nature to the polypeptide 3 (SEQ ID NO: 7) and its foreseeable effect. In this assay, ammonia is released from a reaction in which FXIIIa links a peptide substrate to a glycine ethyl ester in normal pooled plasma (NPP). Ammonia is converted into glutamate by glutamate dehydrogenase upon consumption of NADH that results in a reduction in absorbance. The decrease observed for untreated NPP was measured relative to NPP treated with different inhibitor concentrations and the effect of the tridegin analogs was normalized to the effect of lead polypeptide isomer B (SEQ ID NO: 6) as described earlier (Fig. 2C). Polypeptides 2 (SEQ ID NO: 2) and 3 (SEQ ID NO: 7) showed a lower level of inhibition (81.2 ± 0.3%, n = 4 and 51.4 ± 0.2%, n = 4, respectively) than polypeptide isomer B (SEQ ID NO: 6), which is in agreement with the activity reduction that was observed in the fluorogenic enzyme assay. However, only approx. 20% FXIIIa inhibition was achieved at a concentration of 1 μM for polypeptide isomer B (SEQ ID NO: 6) in NPP (Fig. 2D). For complete inactivation higher compound concentrations were required (>100 μM). A continuous reduction was observed at increasing inhibitor concentrations (1-100 μM) for all three compounds investigated while ratios between polypeptides isomer B (SEQ ID NO: 6), 2 (SEQ ID NO: 2), and 3 (SEQ ID NO: 7) remained unaffected (Fig. 2D).

Additionally, inhibition of FXIIIa was determined in human and mouse whole blood by measuring the clot size in a whole blood contraction assay as performed earlier. Red blood cell retention (promoted by FXIIIa) is inhibited by polypeptides isomer B (SEQ ID NO: 6) and 3 (SEQ ID NO: 7) in equipotent amounts in human blood with an $IC_{50}$ between 1-5 μM and, therefore, whole blood clot size and weight are reduced (Fig. 2E). At the same time a slightly more potent inhibition of the clot weight formation was observed when polypeptide isomer B (SEQ ID NO: 6) and 3 (SEQ ID NO: 7) were added to whole blood from mice ($IC_{50}$: 2.2 ± 0.8 μM with n = 3 and 2 μM with n = 1, respectively).

Thus, the effects observed for the linear polypeptide 3 (SEQ ID NO: 7) and oxidized tridegin with two disulfide bridges ($Cys^5$-$Cys^{37}$, $Cys^{17}$-$Cys^{31}$) of isomer B (SEQ ID NO: 6) are in line with the aforementioned findings of the fluorogenic assay. The slight drop in activity of the linear polypeptides may not be reflected to the same extent by the whole blood assays as compared to the isolated *in vitro* FXIIIa assays. This is probably due to the more complex environment that may compensate these slight differences. In order to figure out which impact the environment in the blood may have on the inhibitory effect, i.e., how pharmacokinetic parameters such as plasma protein binding or metabolization by liver microsomes may be affected, ADME-T studies have been conducted.

Pharmacokinetics of tridegin derivatives

The above-referenced six polypeptides were subjected to pharmacokinetic studies to validate their early-stage potential as future drugs as follows in Table 2.

Table 2. *In vitro* analysis for ADME-T properties of polypeptides isomer B (SEQ ID NO: 6) and 3 (SEQ ID NO: 7) are shown as representatives for all peptides investigated in this study.

| Poly pepti de | Solubility (mg/ml)[a] | Log $D_{7.4}$ | Plasma stability (3 h) | Plasma protein binding (%) | Hepatic microsome stability (1 h) | Cytotoxic ity ($LC_{50}$, μM)[b] |
|---|---|---|---|---|---|---|
| **1** | > 100 | << 0 | stable | > 99 | stable | > 100 |
| **3** | > 100 | << 0 | stable | > 99 | stable | > 100 |
| [a] measured in 0.1 M phosphate buffer, pH7.4.<br>[b] tested in an MTT-based assay on HepG2 cells. | | | | | | |

Accordingly, proper assessment of parameters such as aqueous solubility, hydrophobicity, stability, and toxicity are essential key criteria. All polypeptides showed solubility in aqueous media (phosphate buffers at pH 7.4, 6.2 and 5.0) above 100 μM combined with a high hydrophilicity (Log $D_{7.4}$ << 0).

Stability upon application and incubation in plasma or with hepatic microsomes was suitable, i.e., no degradation or modification was observed in the studied time range.

Also, no compound showed cytotoxic effects towards HepG2 cells below 100 μM ($LC_{50}$ > 100 μM), whereas we observed plasma protein binding to be > 99 % (Table 2).

Behavior of all analogs of tridegin was comparable in our pharmacokinetic studies and for representation

polypeptides isomer B (SEQ ID NO: 6) and 3 (SEQ ID NO: 7) have been shown in detail in Table 2.

In addition to the aforementioned *in vitro* pharmacokinetics, we also conducted *in vivo* stability studies for polypeptide isomer B (SEQ ID NO: 6) in mice. After retro-orbital injection of the compound (20 μM) blood of the treated mice was drawn from the inferior vena cava at defined times and clotting properties were evaluated by measuring the clot mass that was formed in the whole blood contraction assay. While up to 2 min a clear inhibition of FXIIIa could be detected, at 15 min no further effect on the clot mass was observed (Figure 3).

Consequently, these results align with the observation of high plasma protein binding that most likely leads to fast elimination of the polypeptide from the mice.

## Claims

**1.** A polypeptide comprising a polypeptide strand of SEQ ID NO: 1:

$$X^1LLPX^5X^6EWHQGIPNPX^{16}X^{17}WX^{19}GADLEX^{25}AQDQYX^{31}AFIPQX^{37}X^{38}PX^{40}SELIX^{45}PMDDIYQX^{53}PVEFPNLPLX^{63}PX^{65}E,$$

wherein amino acid moieties $X^{19}$ and $X^{25}$ are each independently selected from the group consisting of Ala and Cys(Me);
wherein amino acid moieties $X^1$, $X^6$, $X^{16}$, $X^{38}$, $X^{40}$, $X^{45}$, $X^{53}$, $X^{63}$, and $X^{65}$ are each independently selected from the group consisting of Lys, D-Lys, Arg, D-Arg, Orn, and Cit;
wherein amino acid moieties $X^5$, $X^{17}$, $X^{31}$, and $X^{37}$ are each independently selected from the group consisting of Ser, Ala, Cys(Me), an amino acid moiety forming a lactam bond with another one of the amino acid moieties selected from the group consisting of $X^5$, $X^{17}$, $X^{31}$, and $X^{37}$, and Cys optionally forming a disulfide bond with another Cys at an amino acid moiety selected from the group consisting of $X^5$, $X^{17}$, $X^{31}$, and $X^{37}$;
wherein not more than two of $X^5$, $X^{17}X^{31}$, and $X^{37}$ are Cys,
wherein optionally one or more of the L-amino acid moieties may be replaced by the respective one or more D-amino acids,
or a retro-inverso analogue of the sequence thereof,
or a peptidomimetic analogue of the sequence thereof,
or a pharmaceutical salt thereof.

**2.** The polypeptide of claim 1, wherein at least two of $X^5$, $X^{17}$, $X^{31}$, and $X^{37}$ are each independently selected from the group consisting of Cys(Me), Ser and Ala, preferably wherein all of $X^5$, $X^{17}$, $X^{31}$, and $X^{37}$ are each independently selected from the group consisting of Cys(Me), Ser and Ala.

**3.** The polypeptide of any one of claims 1 or 2, wherein the N-terminus of polypeptide strand is:

(i) acetylated;
(ii) an unbound primary amino group;
(iii) conjugated to a peptidic moiety, a non-peptidic moiety or a moiety comprising peptidic and non-peptidic structure elements, of a total molecular weight of not more than 500 Da,
(iv) conjugated to a peptidic polymeric structure, a non-peptidic polymeric structure or a polymeric structure comprising peptidic and non-peptidic structure elements, of a total molecular weight of more than 500 Da, preferably of 500 to 100,000 Da; or
(v) conjugated to solid support, optionally via a peptidic linker structure, a non-peptidic linker structure or a linker structure comprising peptidic and non-peptidic structure elements, preferably wherein the total linker structure has a molecular weight of 100 to 10,000 Da.

**4.** The polypeptide of any one of claims 1 to 3, wherein the C-terminus of polypeptide strand is:

(i) amidated,
(ii) an unbound carboxylic group;
(iii) conjugated to a peptidic moiety, a non-peptidic moiety or a moiety comprising peptidic and non-peptidic structure elements, of a total molecular weight of not more than 500 Da;
(iv) conjugated to a peptidic polymeric structure, a non-peptidic polymeric structure or a polymeric structure

comprising peptidic and non-peptidic structure elements, of a total molecular weight of more than 500 Da, preferably of 500 to 100,000 Da; or
(v) conjugated to solid support, optionally via a peptidic linker structure, a non-peptidic linker structure or a linker structure comprising peptidic and non-peptidic structure elements, preferably wherein the total linker structure has a molecular weight of 100 to 10,000 Da.

**5.** The polypeptide of any one of claims 1 to 4, wherein amino acid moieties $X^5$, $X^{17}$, $X^{31}$, and $X^{37}$ are each not Cys.

**6.** The polypeptide of claim 5, wherein a lactam bond is formed between amino acid moieties $X^5$ and $X^{37}$ and/or a lactam bond is formed between amino acid moieties $X^{17}$ and $X^{31}$, or between amino acid moieties $X^5$ and $X^{17}$ and between amino acid moieties $X^{31}$ and $X^{37}$, or between amino acid moieties $X^5$ and $X^{31}$ and between amino acid moieties $X^{17}$ and $X^{37}$.

**7.** The polypeptide of any one of claims 1 to 6, wherein at least one of the moieties selected from the group consisting of $Lys^6$, $Ile^{12}$, $Leu^{23}$ or $Ile^{34}$ is replaced by the respective D-amino acid residue,
in particular wherein the moieties selected from the group consisting of $Lys^6$, $Ile^{12}$, $Leu^{23}$ or $Ile^{34}$ are each replaced by the respective D-amino acid residues.

**8.** The polypeptide of any one of claims 1 to 7, wherein one or more amino acid moiety positions $X^1$, $X^6$, $X^{45}$ and $X^{63}$ are each independently from each other selected from the group consisting of D-Lys and Orn and/or one or more amino acid moiety positions $X^{16}$, $X^{38}$, $X^{40}$, $X^{53}$, $X^{65}$ are each independently from each other selected from the group consisting of D-Arg and Cit,
in particular wherein amino acid moiety positions $X^1$, $X^6$, $X^{45}$ and $X^{63}$ vD-Lys and Orn and amino acid moiety positions $X^{16}$, $X^{38}$, $X^{40}$, $X^{53}$, $X^{65}$ are each independently from each other selected from the group consisting of D-Arg and Cit.

**9.** The polypeptide of any one of claims 1 to 8, wherein the polypeptide comprises (or consists of) a polypeptide strand selected from the group consisting of

KLLPC*k*EWHQG*i*PNPRCWSGAD*l*ESAQDQYCAF*i*PQCRPRSELIKPMDDIY QRPVEFPNLPLKPRE (SEQ ID NO: 2),

wherein $k^6$ is a D-lysyl moiety, $l^{23}$ is
a D-leucine and $i^{12}$ and $i^{34}$ are a D-isoleucine moiety,

$X^1$LLPS$X^6$EWHQGIPNP$X^{16}$SWSGADLESAQDQYSAFIPQS$X^{38}$P$X^{40}$SELI$X^{45}$ PMDDIYQ$X^{53}$PVEFPNLPL$X^{63}$P$X^{65}$E (SEQ ID NO: 3),

wherein $X^1$, $X^6$, $X^{45}$ and $X^{63}$ are each Orn; and wherein $X^{16}$, $X^{38}$, $X^{40}$, $X^{53}$, $X^{65}$ are each Cit,

KLLPAKEWHQGIPNPRAWAGADLEAAQDQYAAFIPQARPRSELIKPMDDI YQRPVEFPNLPLKPRE (SEQ ID NO: 4),

and

$X^1$LLPA$X^6$EWHQGIPNP$X^{16}$AWAGADLEAAQDQYAAFIPQA$X^{38}$P$X^{40}$SELI$X^{45}$ PMDDIYQ$X^{53}$PVEFPNLPL$X^{63}$P$X^{65}$E (SEQ ID NO: 5),

wherein $X^1$, $X^6$, $X^{45}$ and $X^{63}$ are each Orn; and wherein $X^{16}$, $X^{38}$, $X^{40}$, $X^{53}$, $X^{65}$ are each Cit,
wherein optionally one or more of the L-amino acid moieties may be replaced by the respective one or more D-amino acids,
or a retro-inverso analogue of any one of the sequences,
or a peptidomimetic analogue of any one of the sequences,
or a pharmaceutically acceptable salt of any one of the sequences.

**10.** The polypeptide of any one of claims 1 to 9, wherein the polypeptide does not comprise a disulfide bond, in particular does not comprise an internal bridge or comprises one, two or three lactam bonds between one or more Asp residues and an equivalent number of Lys, Orn or Dap moieties.

**11.** A pharmaceutical composition comprising:

(A) the polypeptide of any one of claims 1 to 10; and
(B) at least one pharmaceutically acceptable carrier.

**12.** The polypeptide of any one of claims 1 to 10 or a pharmaceutical composition of claim 11 for use in a method for treating or preventing a thrombotic event in a patient.

**13.** The polypeptide for use of claim 12, wherein the patient is:

(a) at risk of developing or is suffering from a pathological state associated with a thrombotic event selected from the group consisting of stenosis of one or more veins, venules, arteria, arterioles, and/or capillaries,
(b) at risk of developing or is suffering from a pathological state associated with a thrombotic event selected from the group consisting of stroke, infarction, embolism, disseminated intravascular coagulation (DIC), thrombotic thrombocytopenic purpura (TTP), thrombophlebitis, sepsis, or a combination of two or more thereof; and/or
(c) subjected to a surgery, in particular is receiving an endoprosthesis and/or is receiving an organ transplant and/or a tissue transplant.

**14.** An endoprosthesis or an enzyme-linked immunosorbent assay (ELISA) plate coated with the polypeptide of any one of claims 1 to 10.

**15.** The endoprosthesis of claim 14, wherein the endoprosthesis is selected from the group consisting of a blood vessel endoprosthesis, an artificial joint, a cardiac pacemaker, an artificial bowel outlet, and a cosmetic implant.

Fig. 1A

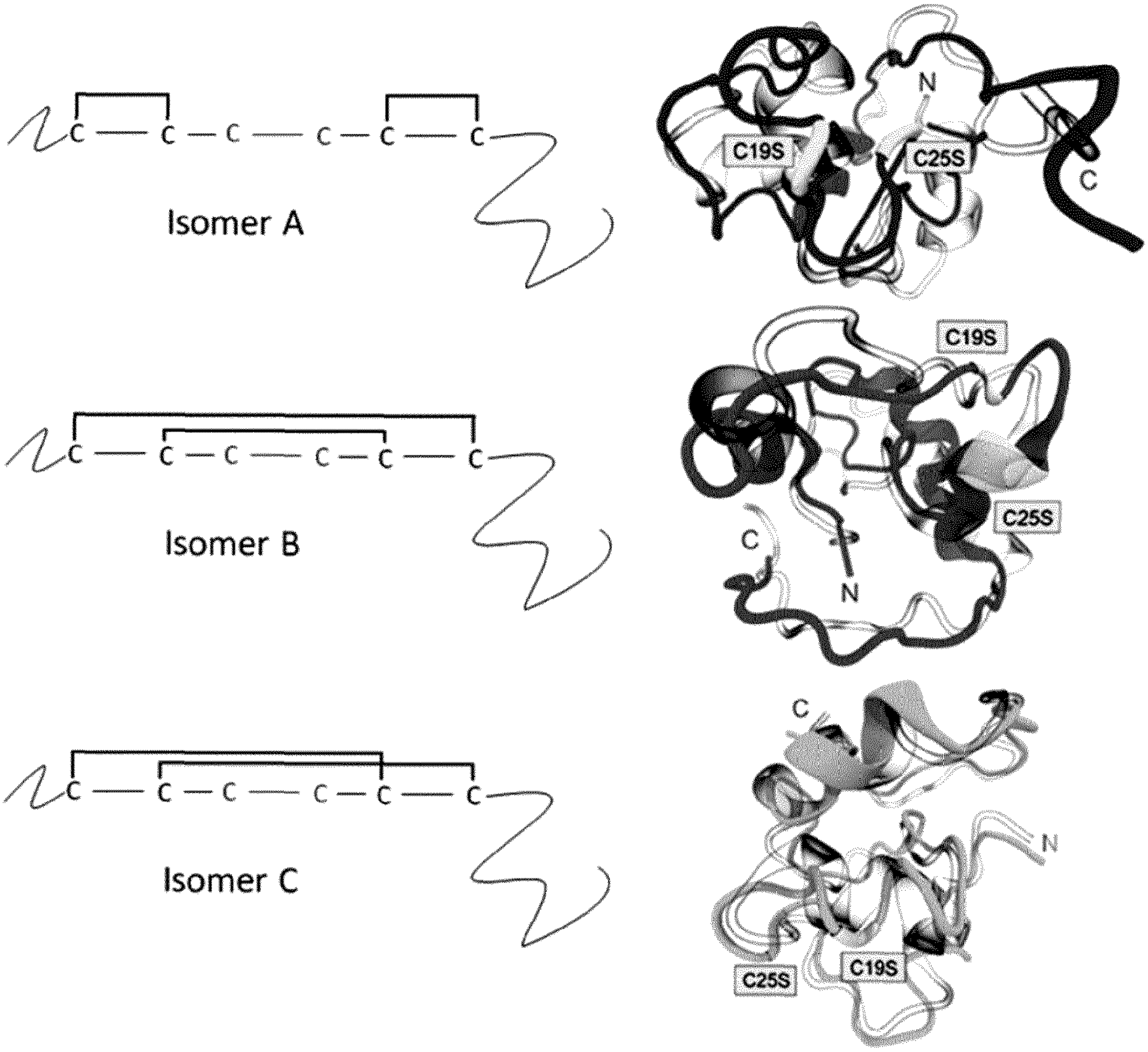
Isomer A
C19S
C25S
N
C
Isomer B
C19S
C25S
C
N
Isomer C
C
N
C25S
C19S

Fig. 1B

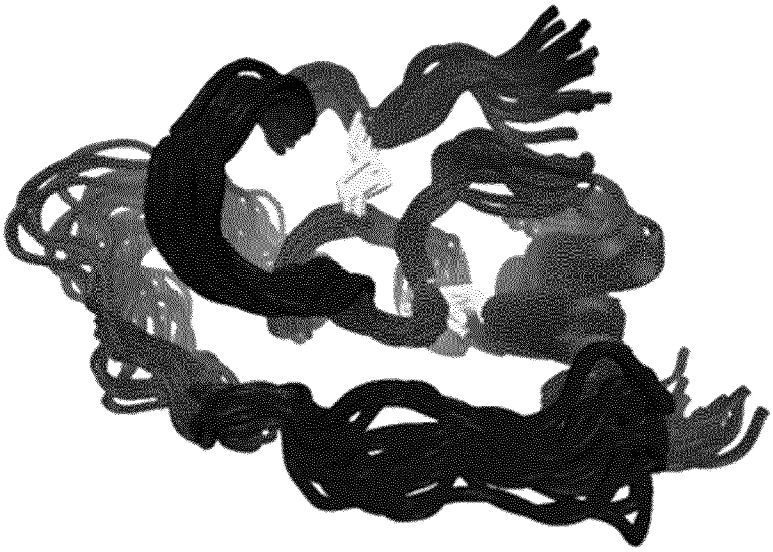

Fig. 1C

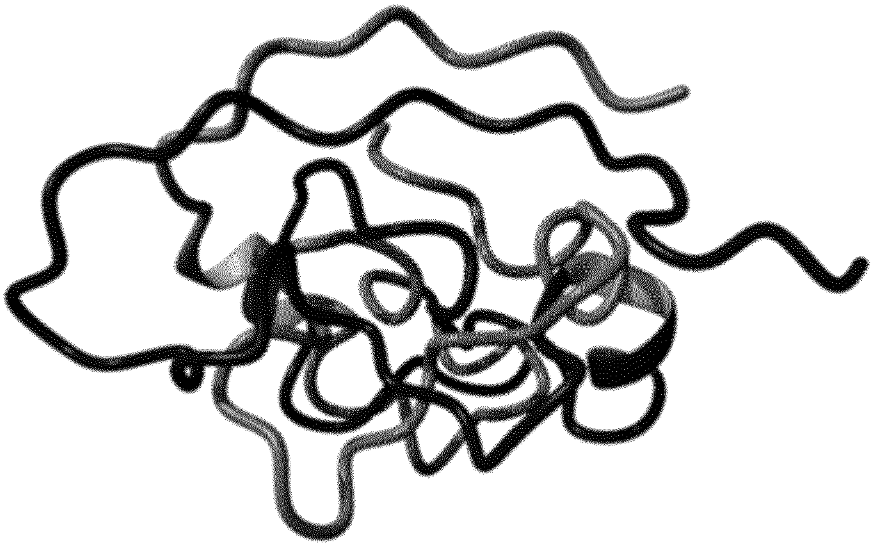

Fig. 2

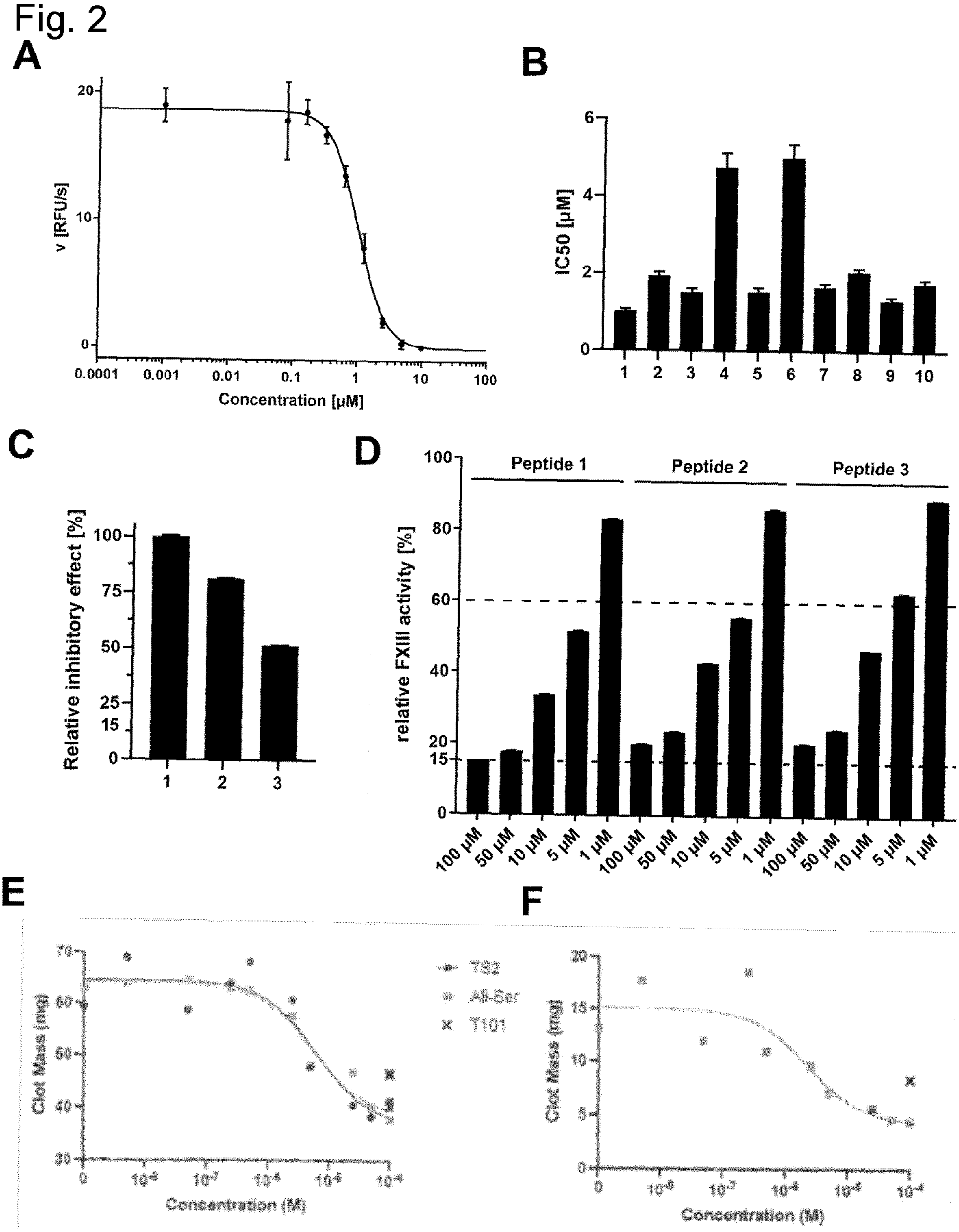
A
20
10
0
v [RFU/s]
0.0001
0.001
0.01
0.1
1
10
100
Concentration [µM]
B
6
4
2
0
IC50 [µM]
1
2
3
4
5
6
7
8
9
10
C
Relative inhibitory effect [%]
100
75
50
25
15
0
1
2
3
D
Peptide 1
Peptide 2
Peptide 3
100
80
60
40
20
15
0
relative FXIII activity [%]
100 µM
50 µM
10 µM
5 µM
1 µM
E
70
60
50
40
30
Clot Mass (mg)
TS2
All-Ser
T101
Concentration (M)
F
20
15
10
5
0
Clot Mass (mg)
Concentration (M)

Fig. 3

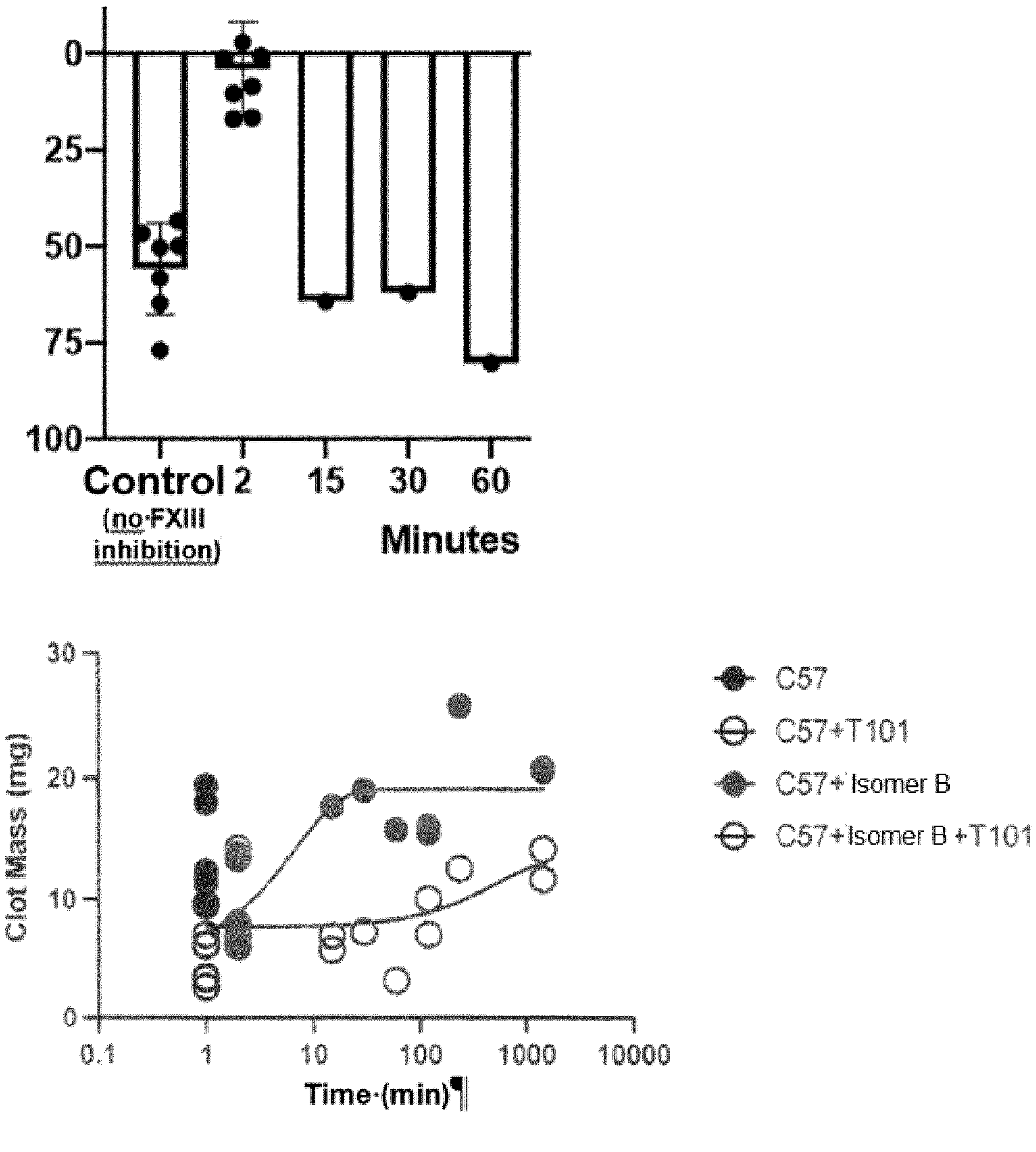
0
25
50
75
100
Control
(no·FXIII
inhibition)
2
15
30
60
Minutes
30
20
10
0
Clot Mass (mg)
0.1
1
10
100
1000
10000
Time·(min)¶
C57
C57+T101
C57+ Isomer B
C57+Isomer B +T101

Europäisches Patentamt
European Patent Office
Office européen des brevets

# EUROPEAN SEARCH REPORT

Application Number
EP 24 19 0365

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | BÄUML C A ET AL: "Coagulation Factor XIIIa Inhibitor Tridegin: On the Role of Disulfide Bonds for Folding, Stability, and Function", JOURNAL OF MEDICINAL CHEMISTRY, vol. 62, no. 7, 11 April 2019 (2019-04-11), pages 3513-3523, XP093237243, DOI: 10.1021/acs.jmedchem.8b01982 Retrieved from the Internet: URL:https://pubs.acs.org/doi/pdf/10.1021/acs.jmedchem.8b01982> * the whole document, in particular abstract; page 3520, paragraph spanning columns; figures 1, 3 * ----- | 1-15 | INV. C07K14/435 A61P7/02 A61K38/17 |
| A | Schmitz Thomas: "Influence of disulfide-bonds on structural and functional properties of peptides and proteins -Case studies on FXIIIa inhibitor tridegin and -conotoxin PIIIA", Dissertation zur Erlangung des Doktorgrades (Dr. rer. nat.) der Mathematisch-Naturwissenschaftlichen Fakultät der Rheinischen Friedrich-Wilhelms-Universität Bonn, 1 February 2021 (2021-02-01), XP093237387, Retrieved from the Internet: URL:https://bonndoc.ulb.uni-bonn.de/xmlui/bitstream/20.500.11811/9110/1/6244.pdf * the whole document, in particular page 84, lines 24-26; paragraph spanning pages 93 and 94 * ----- | 1-15 | TECHNICAL FIELDS SEARCHED (IPC): A61P C07K A61K G01N A61F |

The present search report has been drawn up for all claims

1

| Place of search | Date of completion of the search | Examiner |
|---|---|---|
| Munich | 16 January 2025 | Weber, Peter |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9634890 A
- US 20090226415 A


### Non-patent literature cited in the description


- **FINNEY et al.** *Biochemical Journal*, 1997, vol. 324, 797-805
- **WALLIS et al.** *Blood Coagulation and Fibrinolysis*, 1997, vol. 8, 291-295
- **SEALE et al.** *Thrombosis and Haemostasis*, 1997, vol. 77 (5), 959-963
- **BÖHM et al.** *Journal of Medicinal Chemistry*, 2014, vol. 57, 10355-10365
- **BÄUML et al.** *Journal of Medicinal Chemistry*, 2019, vol. 62, 3513-3523
- **BÄUML et al.** *European Journal of Medicinal Chemistry*, 2020, vol. 201, 112474
- **SCHMITZ et al.** *International Journal of Molecular Sciences*, 2021, vol. 22, 880